(19) Europäisches Patentamt
European Patent Office
Office européen des brevets

(11) **EP 2 351 519 B1**

(12) **EUROPEAN PATENT SPECIFICATION**

(45) Date of publication and mention
of the grant of the patent:
**27.12.2017   Bulletin 2017/52**

(51) Int Cl.:
***A61B 5/0245*** (2006.01)

(21) Application number: **11152666.1**

(22) Date of filing: **31.01.2011**

(54) **Biological information measuring device**

Vorrichtung zur Messung biologischer Informationen

Dispositif de mesure d'informations biologiques

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.02.2010   JP 2010020015
29.09.2010   JP 2010218337**

(43) Date of publication of application:
**03.08.2011   Bulletin 2011/31**

(73) Proprietor: **Seiko Epson Corporation
Shinjuku-ku
Tokyo (JP)**

(72) Inventors:
• **Kato, Hidetada
Nagano 392-8502 (JP)**
• **Shiho, Kazuhiro
Nagano 392-8502 (JP)**
• **Heishi, Toshihiro
Nagano 392-8502 (JP)**
• **Nakazawa, Hironori
Nagano 392-8502 (JP)**

(74) Representative: **Hoffmann Eitle
Patent- und Rechtsanwälte PartmbB
Arabellastraße 30
81925 München (DE)**

(56) References cited:
**US-A- 5 795 301          US-A1- 2002 107 451
US-A1- 2004 122 333     US-A1- 2007 159 926
US-A1- 2009 054 751**

## Description

## BACKGROUND

**Technological Field**

The present invention relates to a biological information measuring device and similar devices.

**Background Technology**

The intensity of an exercise when, e.g., a person goes for a walk, can be represented by a heart rate (or in a broader sense, biological information). The exercise intensity of such description can be evaluated using an evaluation formula such as the Karvonen method. Since the heart rate normally matches the pulse rate (or in a broader sense, biological information), the pulse rate can be used instead of the heart rate. Specifically, a pulse rate monitor, which is an example of a biological information measuring device that measures the pulse rate, is sometimes called a heart rate monitor. The exercise intensity may also be evaluated according to, e.g., oxygen intake, blood lactate concentration, and other biological information instead of the pulse rate or the heart rate. Specifically, oxygen intake, blood lactate concentration, and other biological information can be used as biological information that can be converted to exercise intensity.

Japanese Patent No. 3421738 (Patent Citation 1) is an example of the related art.

In Patent Citation 1, there is disclosed a device that notifies a user (or in a broader sense, a test subject) whether the heart rate (or in a broader sense, the exercise intensity) during exercise is within a suitable range (or in a broader sense, a zone) for the heart rate. Although the heart rate is used in Patent Citation 1, a problem is presented in that there are significant individual differences in the rate of increase in heart rate, the rate of decrease in heart rate, and other actual physical performance. Therefore, even on an assumption of the age (or in a broader sense, a biological information parameter) being identical, it is difficult to set a suitable range for the heart rate using a fixed evaluation formula such as that shown in [Mathematical Formula 1] in Claim 1 of Patent Citation 1. For example, in an instance in which a lower threshold (or in a broader sense, a target value) of the suitable range for the heart rate is set high, it is difficult for the heart rate during exercise to reach the suitable range for the heart rate. A situation of such description hinders motivation for exercise.

US 2004/0122333 A1 discloses a method and arrangement for measuring heart rate, including: means for inputting a heart rate limit for an exercise; means for measuring the user's heart rate during the exercise. The arrangement further comprises means for changing the heart rate limit during the exercise on the basis of a predetermined change criterion associated with the exercise.

## SUMMARY

According to an aspect of the invention, there is provided a biological information measuring device as set out in Claim 1.

According to embodiments of the invention, it is possible to provide a biological information measuring device capable of providing motivation for exercise.

## BRIEF DESCRIPTION OF THE DRAWINGS

**Brief Description of the Drawings**

FIG. 1 is an example of a configuration of a biological information measuring device according to a present embodiment;
FIG. 2 is an example of a specific configuration of the biological information measuring device according to FIG. 1;
FIG. 3 is an example of a relationship between age and the resting pulse rate;
FIG. 4 is an example of another specific configuration of the biological information measuring device according to FIG. 1;
FIG. 5 is a flow chart showing an example of operation of the measurement part in FIGS. 2 or 4;
FIG. 6 is an example of a change in pulse rate during a six-minute walk;
FIG. 7 is a flow chart showing an example of operation of the setting part in FIG. 4;
FIG. 8 is an example of pulse rate target value settings according to age;

FIG. 9 is a flow chart showing an example of operation of the correction part in FIG. 4;

FIG. 10 is an example of pulse rate target value settings according to the first measured value;

FIG. 11 is an example of a modification of the specific configuration in FIG. 2;

FIG. 12 is an example of a modification of the specific configuration in FIG. 4;

FIGS. 13(A), 13(B), and 13(C) are other examples of pulse rate target value settings according to the first measured value;

FIG. 14 is an example of zone settings according to the first measured value;

FIG. 15 is another example of the biological information measuring device according to the present embodiment;

FIGS. 16(A) and 16(B) are examples of a notification of a current position within a zone relating to exercise intensity;

FIGS. 17(A), 17(B), 17(C), and 17(D) are examples of a division of zones relating to exercise intensity;

FIGS. 18(A), 18(B), 18(C), 18(D), and 18(E) are examples of a notification of the second measured value;

FIG. 19 is an example of a specific configuration of the biological information measuring device in FIG. 15;

FIG. 20 is a flow chart showing an example of operation of the measurement part in FIG. 19; and

FIG. 21 is a flow chart showing an example of operation of the notification part in FIG. 19.

## DETAILED DESCRIPTION OF EXEMPLARY EMBODIMENTS

A description shall now be given for the present embodiment. The present embodiment described below is not intended to unduly limit the scope of the Claims of the present embodiment. Not every configuration described in the present embodiment is necessarily an indispensible constituent feature of the invention.

### 1. First mode (First exercise period)

### 1.1 Basic configuration

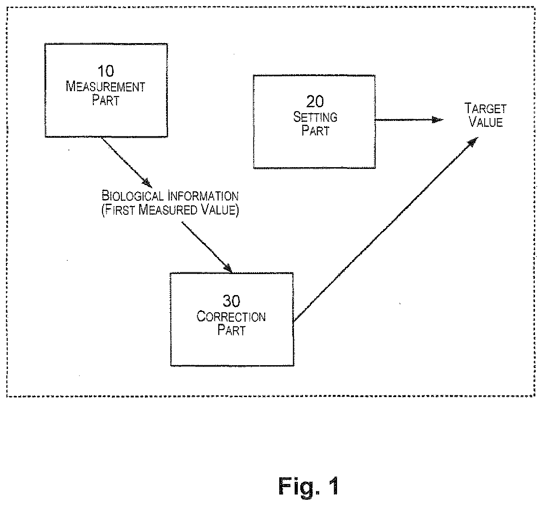

FIG. 1 shows an example of a configuration of the biological information measuring device according to a present embodiment. The example shown in FIG. 1 is an example of configuration corresponding to a first exercise period (or in a broader sense, a first mode), and an example of configurations corresponding to a second exercise period (or in a broader sense, a second mode) that follows the first exercise period will be described further below. As shown in FIG. 1, the biological information measuring device includes a measurement part 10, a setting part 20, and a correction part 30. The measurement part 10 measures biological information. The biological information is, e.g., a pulse rate, and a biological information measuring device including a measurement part 10 for measuring the pulse rate maybe called a pulse rate monitor. The measurement part 10 may also measure the heart rate as with e.g., Patent Citation 1, or may measure oxygen intake, blood lactate concentration, or other biological information that can be converted to exercise intensity.

The setting part 20 sets a target value relating to exercise intensity. A unit for the target value may be the exercise intensity itself, or may be the biological information measured by the measurement part 10 (e.g., the pulse rate). In relation to walking or performing another exercise for lifestyle improvement towards maintaining or improving health, exercise intensity within a range of, e.g., 40% to 70% is known to correspond to aerobic exercise. Therefore, the setting part 20 may set, e.g., 40%, which is a lower limit of a zone in which the exercise intensity ranges from 40% to 70%, as a target value.

Since the exercise intensity may also be represented by biological information that can be converted to exercise intensity using the Karvonen method or another evaluation formula, the setting part 20 may set a pulse rate (e.g., 111 bpm) corresponding to the lower limit of the zone (e.g., 40%) as the target value. The target value relating to exercise intensity may also apply to exercise other than aerobic exercise, and the target value relating to exercise intensity may be an upper limit of the zone, or may be the zone itself.

The correction part 30 corrects the target value (e.g., 111 bpm = 40%) according to a first measured value relating to exercise intensity determined from biological information during the first exercise period. A unit for the first measured value may be the exercise intensity itself, or may be the biological information measured by the measurement part 10 (e.g., the pulse rate). For example, the pulse rate of a test subject (e.g., user A), during an exercise period of, e.g., a six-minute walk, is measured. In an instance in which the biological information during the first exercise period is, e.g., 108 bpm, the first measured value is, e.g., 108 bpm. Also, in an instance in which the exercise intensity corresponding to 108 bpm is, e.g., 37%, the first measured value may be, e.g., 37% (= 108 bpm). The correction part 30 is capable of taking the actual physical performance of the test subject during the first exercise period (e.g., 108 bpm = 37%) into account and correcting the target value (e.g., 111 bpm = 40%).

The user A may, e.g., find it easier to reach a pulse rate of 108 bpm while finding it more difficult to reach an initial target value of 111 bpm. In other words, even in an instance in which the exercise performed by the test subject does not strictly reach the initial target value of 111 bpm, exercise corresponding to a pulse rate of, e.g., 108 bpm can be judged as a "pass" as long as an aim of improving lifestyle towards maintaining or improving health is being met. In such an instance, it is possible to provide the user A with a biological information measuring device (or in a narrower sense, a pulse rate monitor) capable of providing motivation for exercise.

Specifically, in an instance in which the first measured value (e.g., 108 bpm = 37%) is smaller than a first threshold value (e.g., 111 bpm = 40%), the correction part 30 may correct the target value (e.g., 111 bpm = 40%) to the first measured value (e.g., 108 bpm = 37%). The first threshold value is, e.g., the target value; and the unit for the first threshold value may be the exercise intensity itself, or may be the biological information measured by the measurement part 10 (e.g., the pulse rate). For example, the target value may be 111 bpm with the first threshold value being 40%. Using the first threshold value as the target value makes it possible for the biological information for exercise during a second exercise period that follows the first exercise period to readily reach the target value. Therefore, it is possible to provide motivation for exercise.

Also, in an instance in which the biological information during the first exercise period of a user B is, e.g., 89 bpm, the first measured value is, e.g., 89 bpm. In an instance in which the first measured value (e.g., 89 bpm = 20%) is equal to or less than a second threshold value (e.g., 100 bpm = 30%), the correction part 30 may correct the target value (e.g., 111 bpm = 40%) to the second threshold value (e.g., 100 bpm = 30%). The second threshold value may be set so as to be smaller than, e.g., the first threshold value. The unit for the second threshold value may be the exercise intensity itself, or may be the biological information measured by the measurement part 10 (e.g., the pulse rate). Using the second threshold value as the target value makes it possible to prevent a low target value from being set. In other words, even in an instance in which the user B is readily capable of reaching a pulse rate of, e.g., 89 bpm, it can be judged as a "fail" if the aim of improving lifestyle towards maintaining or improving health is not being met. However, judging that reaches the second threshold value (e.g., 100 bpm = 30%) to be a "pass" makes it possible to make the user B motivated for exercise compared to an instance in which it is judged to be a "pass" upon reaching the original 111 bpm (=40%).

While in the example shown in FIG. 1, the biological information measuring device includes the measurement part 10, the setting part 20, and the correction part 30; each of the measurement part 10, the setting part 20, and the correction part 30 may include, e.g., a gate array or another ASIC. All or a part of the measurement part 10, the setting part 20, and the correction part 30 may include a computer (or in a broader sense, a device including a processing part and a memory part). The processing part is, e.g., an MPU (i.e., microprocessing unit). The memory part functions as a work region of the processing part (or in a narrower sense, all or some of the measurement part 10, the setting part 20, and the correction part 30), and the memory part is, e.g., a memory device or a HDD (hard disk drive). The biological information measuring device may have an information storage medium (i.e., a medium that can be read by a computer) for storing, e.g., a program, data, or other information. The information storage medium is, e.g., a memory card or an optical disk. The processing part is capable of performing a variety of processes according to a program stored in the information storage medium or the memory part. Specifically, a program for causing the computer to function as all or some of the measurement part 10, the setting part 20, or the correction part 30 may be stored in the information storage medium or the memory part.

### 1.2 First example of configuration

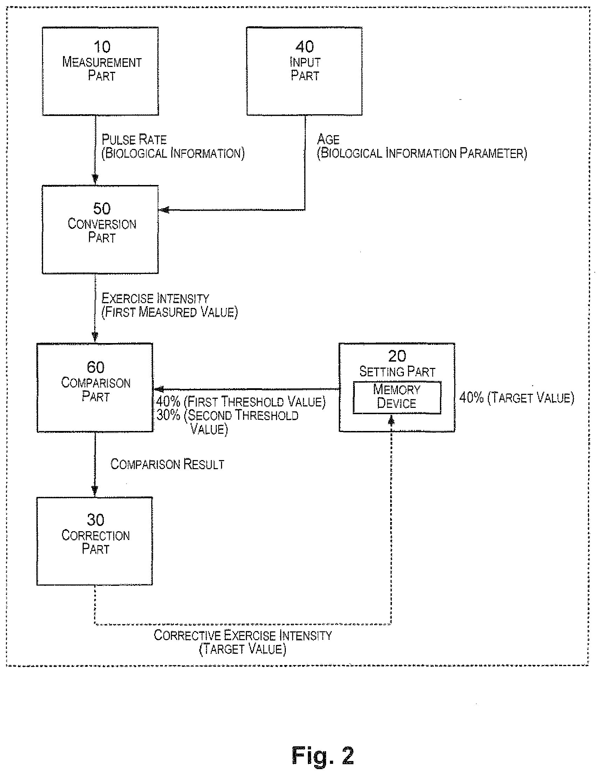

FIG. 2 shows an example of a specific configuration of the biological information measuring device in FIG. 1. In the example shown in FIG. 2, the biological information measuring device is a pulse rate monitor; however, an example of a specific configuration of the biological information measuring device is not limited to that shown in FIG. 2. Structures that are identical to those in the example described above are affixed with the same numerals, and a description of the structures is not provided.

As shown in FIG. 2, the biological information measuring device may further include an input part 40, a conversion part 50, and a comparison part 60. The input part 40 is, e.g., an operation button or a touch panel. The conversion part 50 and the comparison part 60 may include, e.g., a gate array or another ASIC, as with the measurement part 10, the setting part 20, and the correction part 30. All or some of the functions of conversion part 50 and the comparison part 60 may also be performed by at least one of the measurement part 10, the setting part 20, and the correction part 30.

In the example shown in FIG. 2, e.g., age is entered into the input part 40 as a previously known biological information parameter. The conversion part 50 converts the pulse rate from the measurement part 10 into exercise intensity. The conversion part 50 may have a formula for conversion between the pulse rate and exercise intensity, or may have a LUT (i.e., a look-up table) for storing results of conversions between the pulse rate and exercise intensity. The Karvonen method or another evaluation formula may be used as the conversion formula. The Karvonen method can be represented using a following Equation (1).
[Mathematical Formula 1]

$$S = \frac{HR_{AVE} - HR_{REST}}{HR_{MAX} - HR_{REST}} \times 100 \tag{1}$$

In Equation 1, S represents exercise intensity (%), $HR_{AVE}$ represents the pulse rate during exercise (bpm), $HR_{REST}$ represents the resting pulse rate (bpm), and $HR_{MAX}$ represents the maximum pulse rate (bpm).

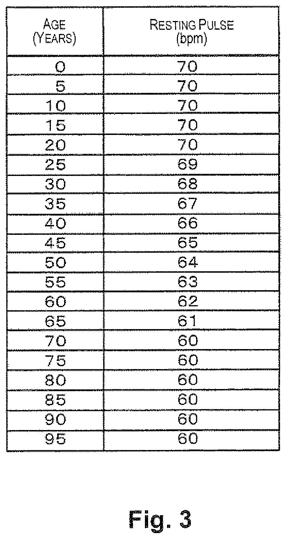

FIG. 3 shows an example of a relationship between age and resting pulse rate. As shown in FIG. 3, the resting pulse rate $HR_{REST}$ can be determined from, e.g., age. For example, in an instance in which the age is 20 years, the resting pulse rate $HR_{REST}$ can be set to 70 bpm. In an instance in which the age is 60 years, the resting pulse rate $HR_{REST}$ can be set to 62 bpm. The resting pulse rate $HR_{REST}$ may be measured using the measurement part 10, or a constant value may be used irrespective of age.

The maximum pulse rate $HR_{MAX}$ is also determined from, e.g., age. However, a constant value may also be used irrespective of age. Since it is difficult to use the measurement part 10 to measure the maximum pulse rate $HR_{MAX}$, the maximum pulse rate $HR_{MAX}$ can be represented using the following Equation 2.
[Mathematical Formula 2]

$$HR_{MAX} = 220 - AGE \tag{2}$$

In Equation 2, "AGE" represents the age in years. In an instance in which, e.g., the age is 20 years, the maximum pulse rate $HR_{MAX}$ can be set to 200 bpm. In an instance in which the age is 60 years, the maximum pulse rate $HR_{MAX}$ can be set to 160 bpm.

In the example shown in FIG. 2, the conversion part 50 is capable of obtaining the exercise intensity according to the pulse rate from the measurement part 10 and the age from the input part 40, using a correspondence relationship such as that shown, e.g., in Equations 1 and 2 and FIG. 3. The exercise intensity obtained in the conversion part 50 is the first measured value (i.e., the actual physical performance of the test subject during the first exercise period). Taking the age from input part 40 into account makes it possible for the biological information measuring device (or in a narrower sense, the correction part 30; or in an even narrower sense, the conversion part 50) to obtain the first measured value in a more accurate manner.

In the example shown in FIG. 2, an exercise intensity of, e.g., 40% is preset in the setting part 20 as the exercise intensity target value. The target value (e.g., an exercise intensity of 40%) can be stored, e.g., in a memory device, and the memory device or another memory part for storing the target value can also be called the setting part 20. In an instance in which the target value (e.g., an exercise intensity of 40%) is the lower limit of the zone, an upper limit of the zone (e.g., an exercise intensity of 70%) may also be preset, as another target value, in the setting part 20.

In the example shown in FIG. 2, a first exercise intensity threshold value of, e.g., 40% is preset in the setting part 20, and a second exercise intensity threshold value of, e.g., 30% is preset in the setting part 20. The comparison part 60 is capable of comparing the first measured value (i.e., the actual physical performance of the test subject during the first exercise period) with at least one of the first threshold value (e.g., an exercise intensity of 40%) and the second threshold value (e.g., an exercise intensity of 30%).

The correction part 30 corrects the target value (e.g., an exercise intensity of 40%) according to a result of the comparison performed by the comparison part 60. In an instance in which the first measured value is smaller than the first threshold value, the correction part 30 corrects the target value to the first measured value (i.e., corrective exercise intensity). In an instance in which the first measured value is equal to or less than the second threshold value, the correction part 30 corrects the target value to the second threshold value (i.e., corrective exercise intensity). In an instance in which the first measured value is equal to or greater than the first threshold value, the correction part 30 may maintain the target value.

### 1.3 Second configuration example

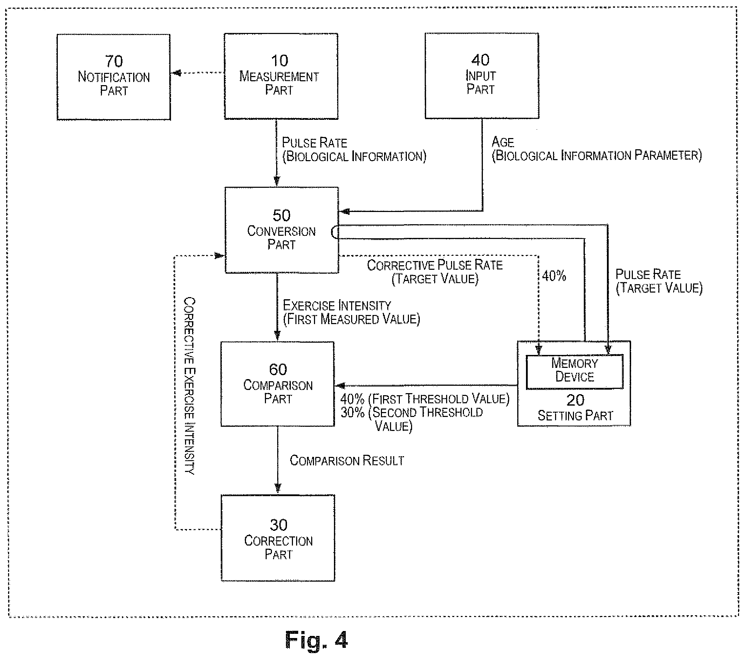

FIG. 4 shows another example of a specific configuration of the biological information measuring device in FIG. 1. Structures that are identical to those in the example described above are affixed with the same numerals, and a description of the structures is not provided. In the example shown in FIG. 2, the exercise intensity target value is preset in the setting part 20, and the exercise intensity target value itself is stored in the memory device. However, in the example shown in FIG. 4, a pulse rate target value is stored in the memory device.

In the example shown in FIG. 4, the conversion part 50 converts the exercise intensity target value (e.g., 40%) from the setting part 20 into a pulse rate target value. Equation 1 described above can be modified into the following Equation 3.
[Mathematical Formula 3]

$$HR_S = \frac{S}{100} \times \left( HR_{MAX} - HR_{REST} \right) + HR_{REST} \tag{3}$$

In Equation 3, $HR_S$ represents the pulse rate target value (bpm), and corresponds to $HR_{AVE}$ in Equation 1. When S = 40% is used as the exercise intensity target value in Equation 3, the pulse rate target value $HR_S$ corresponding to the exercise intensity of 40% can be obtained. For example, in an instance in which the age is 41 years, the pulse rate target value $HR_S$ is 111 bpm. Taking the age from the input part 40 into account makes it possible for the biological information measuring device (or in a narrower sense, the setting part 20, or in an even narrower sense, the conversion part 50) to obtain the pulse rate target value (e.g., 111 bpm) in a more accurate manner. In the example shown in FIG. 4, the target value (e.g., a pulse rate of 111 bpm) can be stored in, e.g., the memory device.

In the example shown in FIG. 2, the correction part 30 directly corrects the target value (e.g., an exercise intensity of 40%). However, in the example shown in FIG. 4, the unit for the target value is the pulse rate; therefore, the correction part 30 corrects the target value (e.g., a pulse rate of 111 bpm) via the conversion part 50. In the example shown in FIG. 4, in an instance in which the first measured value (i.e., the exercise intensity during the first exercise period) is smaller than the first threshold value (e.g., an exercise intensity of 40%), the conversion part 50 converts the first measured value to a pulse rate. The correction part 30 corrects the pulse rate target value stored in the memory device into a pulse rate corresponding to the first measured value (i.e., a corrective pulse rate obtained in the conversion part 50). In an instance in which the first measured value is equal to or less than the second threshold value (e.g., an exercise intensity of 30%), the conversion part 50 converts the second threshold value into a pulse rate. The correction part 30 corrects the pulse rate target value stored in the memory device into a pulse rate corresponding to the second threshold value (i.e., a corrective pulse rate obtained in the conversion part 50).

As shown in FIG. 4, the biological information measuring device includes a notification part 70. The first configuration example shown in FIG. 2 also includes the notification part 70. The notification part 70 (corresponding to a first notification part) is capable of notifying the test subject of an ideal exercise pace for the first exercise period. In order to more accurately obtain the actual physical performance of the test subject during the first exercise period, the biological information measuring device requires the test subject to walk for, e.g., 6 minutes at an exercise pace of, e.g., 2 Hz. For example, the notification part 70 may emit a pacing signal, in the form of a high-pitched alert sound, twice per second. The test subject can form an ideal exercise state by walking forward at a rate of two paces per second in time with the pacing signal. The notification part 70 is, e.g., a speaker, but may also be an LED, a vibrator, or another device. The notification part 70 may emit light twice per second, or may vibrate twice per second.

The notification part 70 also notifies the test subject of a current exercise pace during the first exercise period. The notification part 70 may output a sound, a light, or a similar signal in time with, e.g., the actual walking pace. Obtaining the actual exercise pace makes it possible for the test subject to voluntarily match the actual exercise pace with the ideal exercise pace (e.g., 2 Hz). The walking pace may be measured, e.g., using an acceleration sensor and the walking pace may be measured by, e.g., the measurement part 10. The biological information measuring device is thus capable of notifying the test subject of the ideal exercise pace during the first exercise period and the current pace.

### 1.4 Example of operation

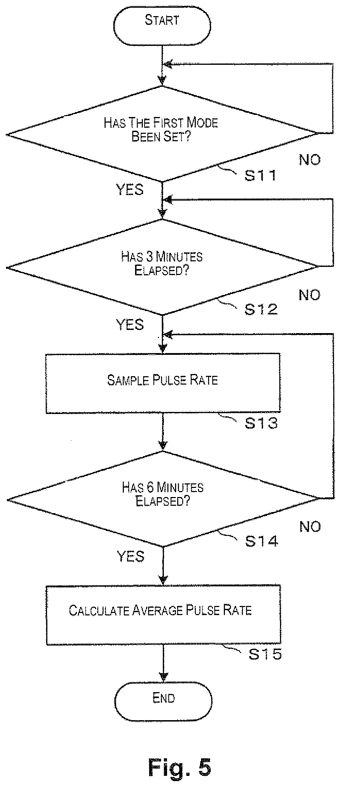

FIG. 5 is a flow chart showing an example of operation of the measurement part 10 shown in FIG. 2 or 4. First, before the measurement part 10 performs the first mode, the test subject prepares for, e.g., a six-minute walk. Specifically, the test subject maintains a state of rest before the six-minute walk, and prepares to set the first mode. Before the first mode is set, the test subject operates the input part 40 and enters an age into the biological information measuring device. The first mode may be set at the same time as the age being entered into the input part 40, or the first mode may be set when a dedicated operation button or a similar input device for setting the first mode is pressed or otherwise operated. For example, the input part 40 may have the dedicated operation button or a similar input device.

In the example shown in FIG. 5, the measurement part 10 (or in a broader sense, the biological information measuring device) judges whether the first mode has been set (step S11). In an instance in which the first mode has not been set, the measurement part 10 performs step S11 again. In an instance in which the first mode has been set, the measurement part 10 performs the first mode. As the first mode, the measurement part 10 measures the time that has elapsed since the first mode has been set.

The test subject sets the biological information measuring device to the first mode, then walks for six minutes. In an instance in which the ideal exercise state is an exercise pace of 2 Hz, the test subject maintains a forward walking rate of two steps a second for six minutes. The notification part 70 can continue issuing a pacing signal in the form of a high-pitched alert sound, emitted twice a second for six minutes, starting at the same time as when the first mode is set. The notification part 70 is also capable of notifying the test subject of the current exercise pace (including the current exercise pace in comparison with the ideal exercise pace) for six minutes according to the actual walking pace, using a message such as "The current exercise pace is 2 Hz," "Please maintain the current pace," "The current exercise pace is 1.9 Hz," "Please walk faster," "The current exercise pace is 2.5 Hz," or "Please walk slower."

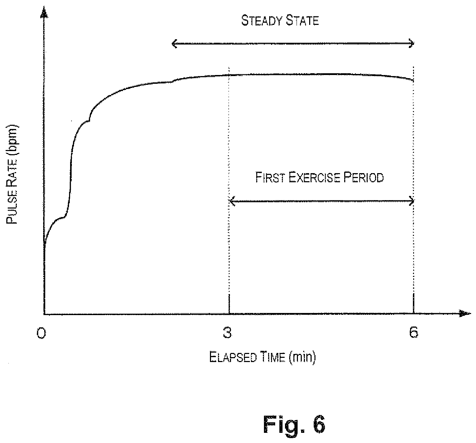

FIG. 6 is an example of a change in pulse rate during a six-minute walk. In the example shown in FIG. 6, when the first mode is set, or specifically, when the elapsed time is 0 minutes, the pulse rate indicates the resting pulse rate. The resting pulse rate when the elapsed time is 0 minutes (i.e., the measured value) may be different from the resting pulse rate determined from age as shown in FIG. 3 (i.e., the estimated value). When the test subject starts walking, the pulse rate rises; subsequently, the pulse rate during exercise indicates a steady state (see FIG. 6). The pulse rate in a steady state can be made to correspond with the actual physical performance of the test subject. Since there are individual differences in a rate of increase in the pulse rate, a range of elapsed time between, e.g., 3 minutes to 6 minutes can be designated as the first exercise period. The measurement part 10 measuring the pulse rate during the first exercise period makes it possible to evaluate the actual physical performance of the test subject. A range of elapsed time between, e.g., 0 minutes to 6 minutes may instead be designated as the first exercise period, and, e.g., the measurement part 10 may determine the presence of a steady state and measure the pulse rate in a steady state during the first exercise period.

In the example shown in FIG. 5, the measurement part 10 (or in a broader sense, the biological information measuring device) judges whether three minutes have elapsed since the first mode has been set (step S12). In an instance in which the elapsed time is less than 3 minutes, the measurement part 10 performs step S12 again. In an instance in which the elapsed time is 3 minutes or above, the measurement part 10 samples the pulse rate at predetermined intervals (step S13). For example, the measurement part 10 measures the pulse rate in real time, and stores the pulse rate, e.g., every 10 seconds.

In the example shown in FIG. 5, the measurement part 10 (or in a broader sense, the biological information measuring device) judges whether six minutes have elapsed since the first mode has been set (step S14). In an instance in which the elapsed time is less than 6 minutes, the measurement part 10 performs step S13 again. In an instance in which the elapsed time is 6 minutes or above, the measurement part 10 discontinues sampling. The measurement part 10 is capable of storing, e.g., 18 (= 3 $\times$ 6) pulse rate values in the range of elapsed time between 3 minutes and 6 minutes.

In the example shown in FIG. 5, the measurement part 10 (or in a broader sense, the biological information measuring device) calculates an average pulse rate (step S15). The measurement part 10 can obtain an average value between, e.g., 18 pulse rate values, and can use the average value as the pulse rate during the first exercise period (or in a broader sense, the biological information). The measurement part 10 may use, instead of the average pulse rate, one of the pulse rates during the steady state.

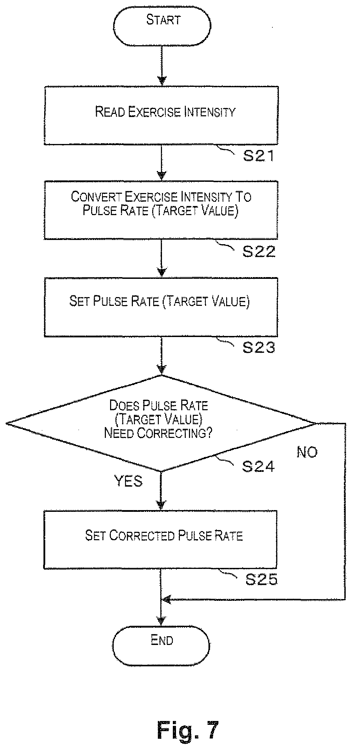

FIG. 7 is a flow chart showing an example of operation of the setting part in FIG. 4. In the example shown in FIG. 7, the setting part 20 (or in a broader sense, the biological information measuring device) reads the preset exercise intensity of, e.g., 40% (step S21). Then the setting part 20 (or in a broader sense, the biological information measuring device) converts the preset exercise intensity (e.g., 40%) to a pulse rate (i.e., target value relating to exercise intensity) according to, e.g., age, e.g., via the conversion part 50 and the input part 40 (step S22). The setting part 20 (or in a broader sense, the biological information measuring device) sets the pulse rate (i.e., target value relating to exercise intensity) to, e.g., the memory device (step S23).

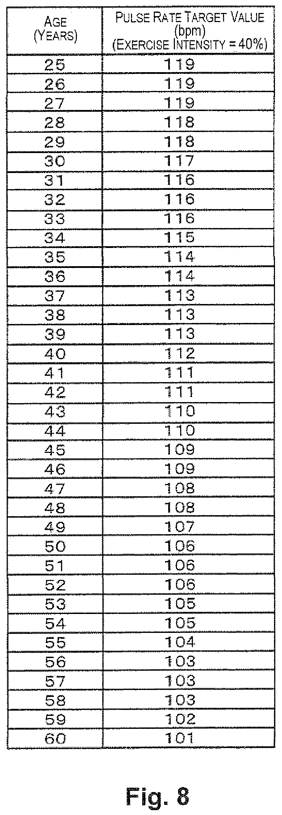

FIG. 8 shows an example of pulse rate target value settings according to age. As shown in FIG. 8, the pulse rate (i.e., target value relating to exercise intensity) can be determined from, e.g., age. A setting relationship such as that shown in FIG. 8 can be determined using a correspondence relationship such as that shown in Equation 1, Equation 2, and FIG. 3. For example, in an instance in which the age of the user A is 41 years, 111 bpm, which corresponds to the preset exercise intensity (e.g., 40%), is set as the pulse rate (i.e., target value relating to exercise intensity) in the memory device (step S23). Also, in an instance in which the age of the user B is also 41 years, 111 bpm is set as the pulse rate (i.e., target value relating to exercise intensity) in the memory device (step S23). In an instance, e.g., where the age of users C and D is 35 years, 114 bpm, which corresponds to the preset exercise intensity (e.g., 40%), is set as the pulse rate (i.e., target value relating to exercise intensity) in the memory device (step S23).

In the example shown in FIG. 7, the setting part 20 (or in a broader sense, the biological information measuring device) judges whether there is a need to correct the pulse rate set in the memory device (i.e., target value relating to exercise intensity), e.g., via the comparison part 60, the correction part 30, and the conversion part 50 (step S24). In an instance in which there is no need to correct the pulse rate (i.e., target value relating to exercise intensity), the setting part 20 maintains the pulse rate (i.e., target value relating to exercise intensity). In an instance in which there is a need to correct the pulse rate (i.e., target value relating to exercise intensity), the setting part 20 overwrites the pulse rate set in the memory device (i.e., target value relating to exercise intensity) with a corrective pulse rate (step S25).

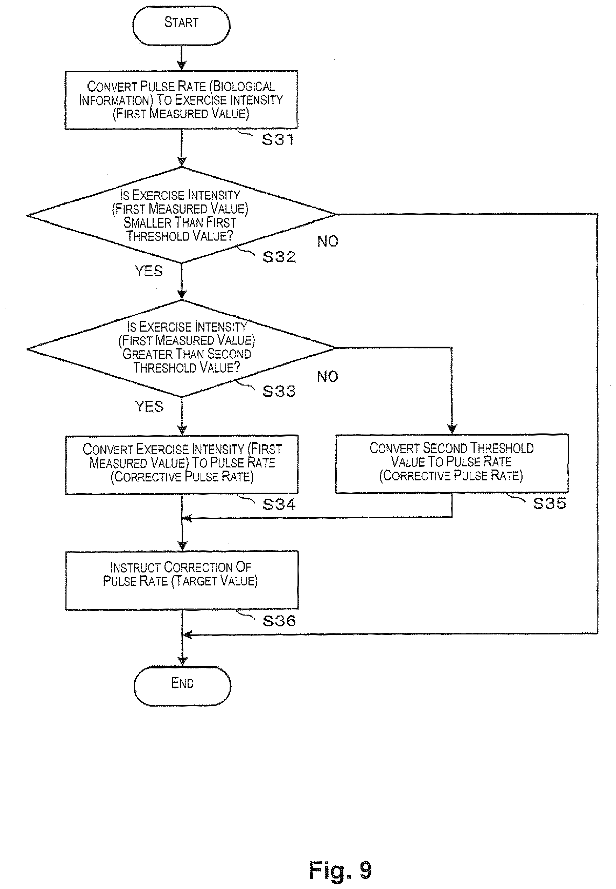

FIG. 9 is a flow chart showing an example of operation of the correction part in FIG. 4. In the example shown in FIG. 9, the correction part 30 (or in a broader sense, the biological information measuring device) converts the pulse rate (or in a broader sense, biological information) during the first exercise period to exercise intensity (i.e., the first measured value) according to, e.g., age, via, e.g., the conversion part 50 and the input part 40 (step S31). The correction part 30 (or in a broader sense, the biological information measuring device) judges, e.g., via the comparison part 60, whether the exercise intensity (i.e., the first measured value) is smaller than the first threshold value (step S32). In an instance in which the exercise intensity (i.e., the first measured value) is smaller than the first threshold value, the correction part 30 (or in a broader sense, the biological information measuring device) further judges whether the exercise intensity (i.e., the first measured value) is greater than the second threshold value (step S33). The first threshold value and the second threshold value are set, e.g., in the setting part 20, and the comparison part 60 (or in a broader sense, the correction part 30) is capable of reading the first threshold value and the second threshold value.

In an instance in which the exercise intensity (i.e., the first measured value) is smaller than the first threshold value and the exercise intensity (i.e., the first measured value) is greater than the second threshold value, the correction part 30 (or in a broader sense, the biological information measuring device) converts the exercise intensity (i.e., the first measured value) to a pulse rate (i.e., corrective pulse rate) according to, e.g., age, e.g., via the conversion part 50 and the input part 40 (step S34). In an instance in which the exercise intensity (i.e., the first measured value) is equal to or less than the second threshold value, the correction part 30 (or in a broader sense, the biological information measuring device) converts the second threshold value to a pulse rate (i.e., corrective pulse rate) according to, e.g., age, e.g., via the conversion part 50 and the input part 40 (step S35).

In the example shown in FIG. 9, the correction part 30 (or in a broader sense, the biological information measuring device) instructs, e.g., the setting part 20 to correct the pulse rate (i.e., the target value), e.g., via the conversion part 50 (step S36). The setting part 20 overwrites the pulse rate set in the memory device (i.e., the target value) using the corrective pulse rate obtained in steps S34 and S35 (step S25 in FIG. 7).

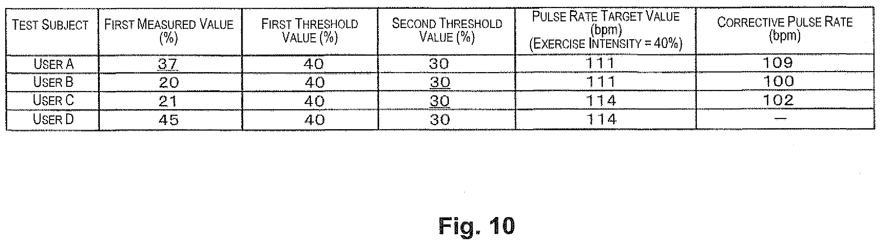

FIG. 10 is an example of pulse rate target value settings according to the first measured value. In the example shown in FIG. 10, in an instance, e.g., where the first measured value for the user A aged 41 years (i.e., an exercise intensity of 37% determined from a pulse rate of 108 bpm during the first exercise period) is smaller than the first threshold value and greater than the second threshold value, 109 bpm, which corresponds to the first measured value, is set as a pulse rate (i.e., target value relating to exercise intensity) in the memory device (steps S34 and S36 in FIG. 9). In an instance, e.g., where the first measured value of the user B aged 41 years (i.e., an exercise intensity of 20% determined from a pulse rate of 89 bpm obtained during the first exercise period) is equal to or smaller than the second threshold value, 100 bpm, which corresponds to the second threshold value, is set as a pulse rate (i.e., target value relating to exercise intensity) in the memory device (steps S35, S36).

In the example shown in FIG. 10, in an instance, e.g., where the first measured value of the user C aged 35 years (i.e., an exercise intensity of 21 % determined from a pulse rate of 92 bpm obtained during the first exercise period) is equal to or smaller than the second threshold value, 102 bpm, which corresponds to the second threshold value, is set as a pulse rate (i.e., target value relating to exercise intensity) in the memory device (steps S35, S36 in FIG. 9). In an instance, e.g., where the first measured value of the user D aged 35 years (i.e., an exercise intensity of 45% determined from a pulse rate of 120 bpm obtained during the first exercise period) is equal to or greater than the first threshold value, 114 bpm, which corresponds to the preset exercise intensity (e.g., 40%), is used without correction.

### 1.5 Example of modification

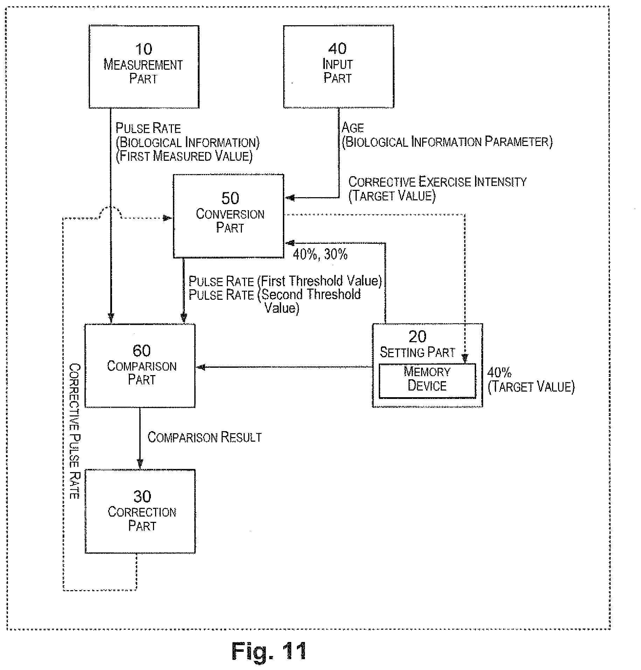

FIG. 11 is an example of a modification of the specific configuration in FIG. 2. Structures that are identical to those in the example described above are affixed with the same numerals, and a description of the structures is not provided. In the example shown in FIG. 2, the exercise intensity and at least one of the first threshold value and the second threshold value are compared by the comparison part 60. In the example shown in FIG. 11, the comparison part 60 uses the pulse rate during the first exercise period itself, instead of the exercise intensity.

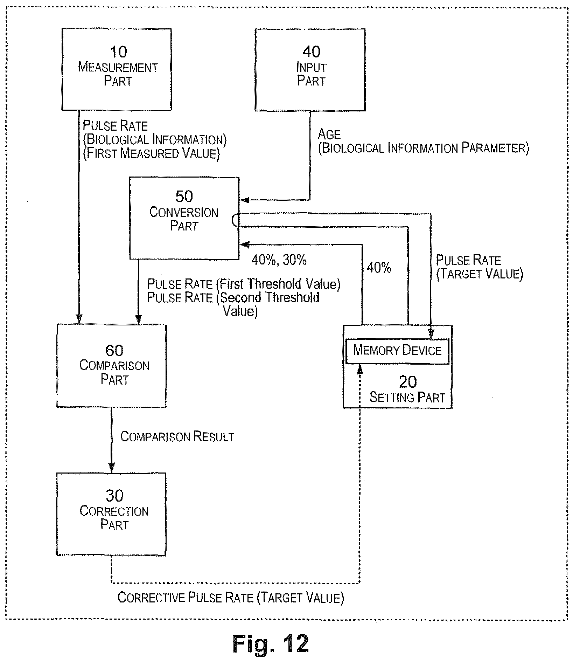

FIG. 12 is an example of a modification of the specific configuration in FIG. 4. Structures that are identical to those in the example described above are affixed with the same numerals, and a description of the structures is not provided. In the example shown in FIG. 4, the exercise intensity and at least one of the first threshold value and the second threshold value are compared by the comparison part 60. In the example shown in FIG. 12, the comparison part 60 uses the pulse rate during the first exercise period itself, instead of the exercise intensity.

In the examples shown in FIGS. 11 and 12, a standard value of the first threshold value of, e.g., 40% is preset in the setting part 20, and a standard value of the second threshold value of, e.g., 30% is preset in the setting part 20. The conversion part 50 (or in a broader sense, the biological information measuring device) converts the preset standard value (e.g., 40%) to a pulse rate (i.e., a first pulse rate threshold value) according to, e.g., age, e.g., via the setting part 20 and the input part 40. The conversion part 50 also converts the preset reference value (e.g., 30%) to a pulse rate (i.e., a second pulse rate threshold value) according to, e.g., age.

In the examples shown in FIGS. 11 and 12, the comparison part 60 is capable of comparing the first measured value (i.e., the pulse rate during the first exercise period itself, relating to exercise intensity) and at least one of the first threshold value (i.e., the pulse rate corresponding to an exercise intensity of, e.g., 40%) and the second threshold value (i.e., the pulse rate corresponding to an exercise intensity of, e.g., 30%).

In the example shown in FIG. 11, the correction part 30 corrects the target value (e.g., exercise intensity = 40%) via the conversion part 50. In the example shown in FIG. 11, in an instance in which the first measured value is smaller than the first threshold value (e.g., a pulse rate corresponding to an exercise intensity of 40%), the conversion part 50 converts the first measured value to exercise intensity. The correction part 30 corrects the exercise intensity target value stored in the memory device into the first measured value (i.e., the corrective exercise intensity obtained in the conversion part 50). In an instance in which the first measured value is equal to or less than the second threshold value (e.g., a pulse rate corresponding to an exercise intensity of 30%), the conversion part 50 converts the second threshold value to exercise intensity. The correction part 30 corrects the pulse rate target value stored in the memory device into the second threshold value (i.e., the corrective exercise intensity obtained in the conversion part 50).

In the example shown in FIG. 12, the correction part 30 corrects the target value (e.g., a pulse rate corresponding to an exercise intensity of 40%) according to a result of the comparison performed by the comparison part 60. In an instance in which the first measured value is less than the first threshold value, the correction part 30 corrects the target value to the first measured value (i.e., corrective pulse rate). In an instance in which the first measured value is equal to or less than the second threshold value, the correction part 30 corrects the target value to the second threshold value (i.e., corrective pulse rate).

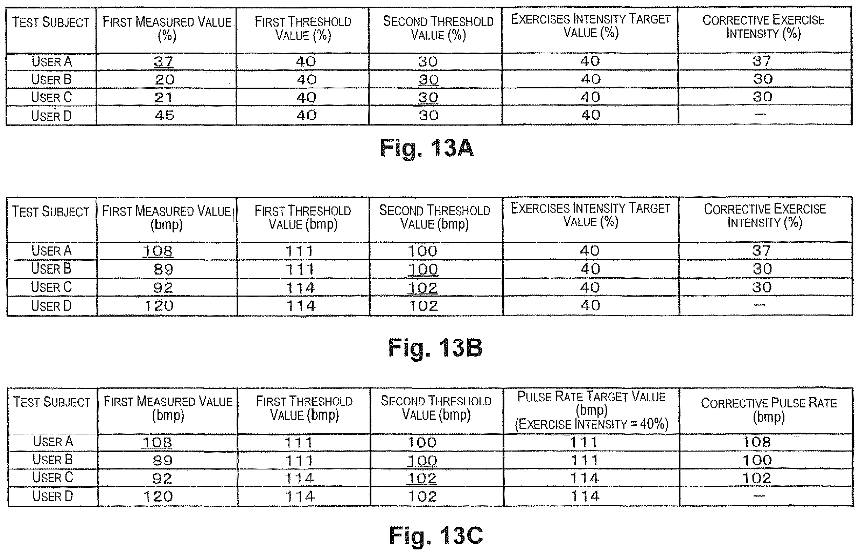

FIGS. 13(A), 13(B), and 13(C) are other examples of pulse rate target value settings according to the first measured value. FIG. 13(A) corresponds to FIG. 2, FIG. 13(B) corresponds to FIG. 11, and FIG. 13(C) corresponds to FIG. 12.

In the example shown in FIG. 13(A), in an instance in which, e.g., the first measured value for the user A aged 41 years (i.e., an exercise intensity of 37% determined from a pulse rate of 108 bpm during the first exercise period) is less than the first threshold value and greater than the second threshold value, the first measured value of 37% is set as the exercise intensity (i.e., target value relating to exercise intensity) in the memory device. In an instance in which, e.g., the first measured value for the user B aged 41 years (i.e., an exercise intensity of 20% determined from a pulse rate of 89 bpm during the first exercise period) is equal to or less than the second threshold value, the second threshold value of 30% is set as the exercise intensity (i.e., target value relating to exercise intensity) in the memory device.

In the example shown in FIG. 13(A), in an instance in which, e.g., the first measured value for the user C aged 35 years (i.e., an exercise intensity of 21% determined from a pulse rate of 92 bpm during the first exercise period) is equal to or less than the second threshold value, the second threshold value of 30% is set as the exercise intensity (i.e., target value relating to exercise intensity) in the memory device. In an instance in which, e.g., the first measured value for the user D aged 35 years (i.e., an exercise intensity of 45% determined from a pulse rate of 120 bpm during the first exercise period) is equal to or greater than the first threshold value, the preset exercise intensity (e.g., 40%) is used without correction.

In the example shown in FIG. 13(B), in an instance in which, e.g., the first measured value for the user A aged 41 years (i.e., a pulse rate of 108 bpm during the first exercise period, relating to exercise intensity) is less than the first threshold value and greater than the second threshold value, 37%, which corresponds to the first measured value, is set as the exercise intensity (i.e., target value relating to exercise intensity) in the memory device. In an instance in which, e.g., the first measured value for user B aged 41 years (i.e., a pulse rate of 89 bpm during the first exercise period, relating to exercise intensity) is equal to or less than the second threshold value, 30%, which corresponds to the second threshold value, is set as the exercise intensity (i.e., target value relating to exercise intensity) in the memory device.

In the example shown in FIG. 13(B), in an instance in which, e.g., the first measured value for the user C aged 35 years (i.e., a pulse rate of 92 bpm during the first exercise period, relating to exercise intensity) is equal to or less than the second threshold value, 30%, which corresponds to the second threshold value, is set as the exercise intensity (i.e., target value relating to exercise intensity) in the memory device. In an instance in which, e.g., the first measured value for the user D aged 35 years (i.e., a pulse rate of 120 bpm during the first exercise period, relating to exercise intensity) is equal to or greater than the first threshold value, the preset exercise intensity (e.g., 40%) is used without correction.

In the example shown in FIG. 13(C), in an instance in which, e.g., the first measured value for the user A aged 41 years (i.e., a pulse rate of 108 bpm during the first exercise period, relating to exercise intensity) is less than the first threshold value and greater than the second threshold value, the first measured value of 108 bpm is set as the pulse rate (i.e., target value relating to exercise intensity) in the memory device. In an instance in which, e.g., the first measured value for the user B aged 41 years (i.e., a pulse rate of 89 bpm during the first exercise period, relating to exercise intensity) is equal to or less than the second threshold value, the second threshold value of 100 bpm is set as the pulse rate (i.e., target value relating to exercise intensity) in the memory device.

In the example shown in FIG. 13(C), in an instance in which, e.g., the first measured value for the user C aged 35 years (i.e., a pulse rate of 92 bpm during the first exercise period, relating to exercise intensity) is equal to or less than the second threshold value, 102 bpm, which corresponds to the second threshold value, is set as the pulse rate (i.e., target value relating to exercise intensity) in the memory device. In an instance in which, e.g., the first measured value for the user D aged 35 years (i.e., a pulse rate of 120 bpm during the first exercise period, relating to exercise intensity) is equal to or greater than the first threshold value, the preset exercise intensity (e.g., 114 bpm, which corresponds to 40%) is used without correction.

## 1.6 Setting of zones

As shown in FIGS. 10, 13(A), 13(B), and 13(C), the unit for the target value relating to exercise intensity set in the setting part 20 may be the exercise intensity itself, or may be a pulse rate corresponding to the exercise intensity (or in a broader sense, biological information measured by the measurement part 10). In FIGS. 2, 4, 11, and 12, an exercise intensity of 40% as an exercise intensity target value, or a pulse rate corresponding to 40% as a pulse rate target value, is preset in the setting part 20.

However, as described above, with regards to the exercise intensity target value, the setting part 20 may be preset not only with a lower limit (i.e., 40%) of a zone within which the exercise intensity ranges, e.g., from 40% to 70%, but also with an upper limit of the zone (i.e., 70%). Similarly, with regards to the heart rate target value, the setting part 20 may be preset not only with a lower limit of the zone (i.e., a pulse rate corresponding to 40%), but also with an upper limit of the zone (i.e., a pulse rate corresponding to 70%).

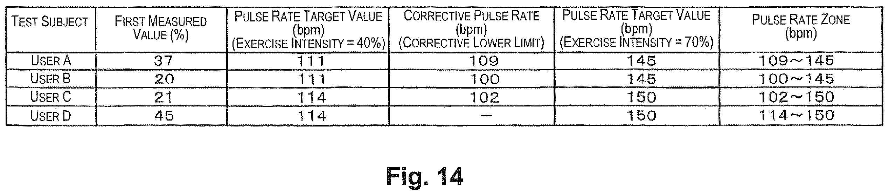

FIG. 14 shows an example of zone settings according to the first measured value. FIG. 14 corresponds to FIG. 10, to which upper limits of zones have been added. In the example shown in FIG. 14, even in an instance in which the lower limit of the zone is corrected according to the first measured value (i.e., the actual physical performance of the test subject during the first exercise period), the upper limit of the zone can be used without correction. Specifically, the zone for the user A aged 41 years is corrected from a range of 111 bpm to 145 bpm to a range of 109 bpm to 145 bpm. The zone for the user B aged 41 years is corrected from a range of 111 bpm to 145 bpm to a range of 100 bpm to 145 bpm. The zone for the user C aged 35 years is corrected from a range of 114 bpm to 150 bpm to a range of 102 bpm to 150 bpm. The zone for the user D aged 35 years has a range of 114 bpm to 150 bpm and remains uncorrected.

## 2. Second mode (Second exercise period that follows the first exercise period).

### 2.1 Basic configuration

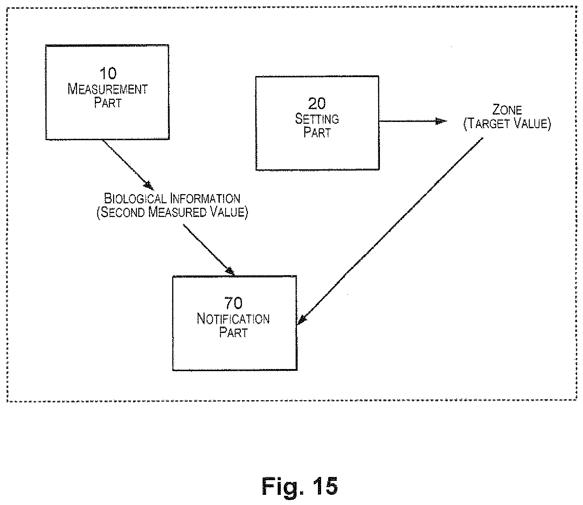

FIG. 15 is another example of the biological information measuring device according to the present embodiment. The example shown in FIG. 15 is an example of a configuration corresponding to a second exercise period (or in a broader sense, a second mode) that follows the first exercise period. The target value relating to exercise intensity that is set in the setting part 20 is corrected as required by the correction part 30 as shown in FIG. 1. Structures that are identical to those in the example described above are affixed with the same numerals, and a description of the structures is not provided.

In the first mode, the target value relating to exercise intensity is set so as to take the first measured value relating to exercise intensity, determined from biological information during the first exercise period, into account. Therefore, after the first exercise period, i.e., after the target value relating to exercise intensity has been appropriately set, the notification part 70 (or in a broader sense, the biological information measuring device) is capable of appropriately evaluating, in the second mode, the exercise state of the test subject performing a walk or another exercise in relation to the zone (i.e., a target value).

In the example shown in FIG. 15, the notification part 70' (corresponding to a second notification part) notifies the test subject of a current position within the zone relating to exercise intensity, according to a second measured value relating to exercise intensity determined from biological information during a second exercise period that follows the first exercise period. The unit for the second measured value may be the exercise intensity itself, or may be the biological information measured by the measurement part 10 (e.g., pulse rate). For example, the pulse rate of a test subject (e.g., user A), during an exercise period of, e.g., a 30 minute walk, is measured. In an instance in which the biological information during the second exercise period (e.g., the pulse rate 10 minutes after the start of a walk) that follows the first exercise period is, e.g., 123 bpm, the first measured value is, e.g., 123 bpm. In an instance in which the exercise intensity corresponding to 123 bpm is, e.g., 50%, the second measured value may be, e.g., 50% (= 123 bpm). The notification part 70 can notify the test subject of an actual physical performance of the test subject during the second exercise period that follows the first exercise period (e.g., a pulse rate of 123 bpm = exercise intensity of 50%).

### 2.2 Example of notification

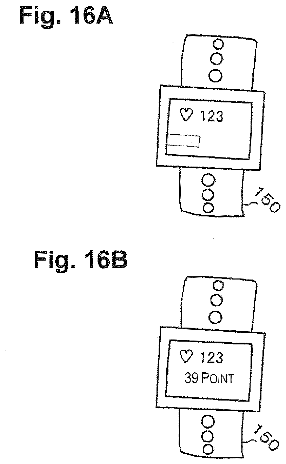

FIGS. 16(A) and 16(B) are examples of a notification of a current position within the zone relating to exercise intensity. FIGS. 16(A) and 16(B) show, e.g., a liquid crystal display or a similar display part as an example of the notification part 70. FIGS. 16(A) and 16(B) also show examples of an outer appearance of the biological information measuring device. The biological information measuring device may further have a wristband 150 capable of attaching the biological information measuring device to an arm (or specifically, a wrist) of the test subject (i.e., the user).

In an instance in which the zone relating to exercise intensity is, e.g., the pulse rate zone of user A in FIG. 14 (i.e., 109 bpm to 145 bpm = 37% to 70%), the notification part 70' can notify the user A of a position of the second measured value (e.g., 123 bpm) within the zone relating to exercise intensity. In the example shown in FIG. 16(A), a current position in the zone relating to exercise intensity is represented using, e.g., a following Equation 4.
[Mathematical Formula 4]

$$X = \frac{Y - Z_{MIN}}{Z_{MAX} - Z_{MIN}}$$

$$(4)$$

In Equation 4, Y represents the second measured value, $Z_{MIN}$ represents the lower limit of the zone, and $Z_{MAX}$ represents the upper limit of the zone. X represents a position in the zone. X in Equation 4 is normalized so that X is equal to 1 when Y is equal to $Z_{MAX}$.

In the example shown in FIG. 16(A), the current position X is displayed as a bar level. In the example shown in FIG. 16(A), the length of a lateral bar shown on the notification part 70 (i.e., the display part) corresponds to the current position X within the zone relating to exercise intensity (= (123 bpm - 109 bpm) / (145 bpm - 109 bpm) = 0.39). The test subject can perceive the length of the bar and thereby obtain the actual physical performance of the test subject during the second exercise period that follows the first exercise period.

In the example shown in FIG. 16(B), the current position within the zone relating to exercise intensity is represented by a score. For example, in an instance in which the second measured value is equal to the lower limit of the zone, a score of zero can be displayed. Also, e.g., in an instance in which the second measured value is equal to the upper limit of the zone, a score of 100 can be displayed. In the example shown in FIG. 16(B), the second measured value (e.g., 123 bpm) is displayed as a score of 39 points. In the example shown in FIG. 16(B), the value of the score corresponds to the current position X in the zone relating to exercise intensity represented by Equation 4 (i.e., 0.39), for example. The test subject can perceive the value of the score and thereby obtain the actual physical performance of the test subject during the second exercise period that follows the first exercise period.

The configuration of the notification part 70' is not limited to that shown in FIGS. 16(A) and 16(B), and the current position in the zone relating to exercise intensity may be outputted, e.g., as an audible message. As shown in FIGS. 16(A) and 16(B), the notification part 70 can display the second measured value (e.g., the current pulse rate of 123 bpm).

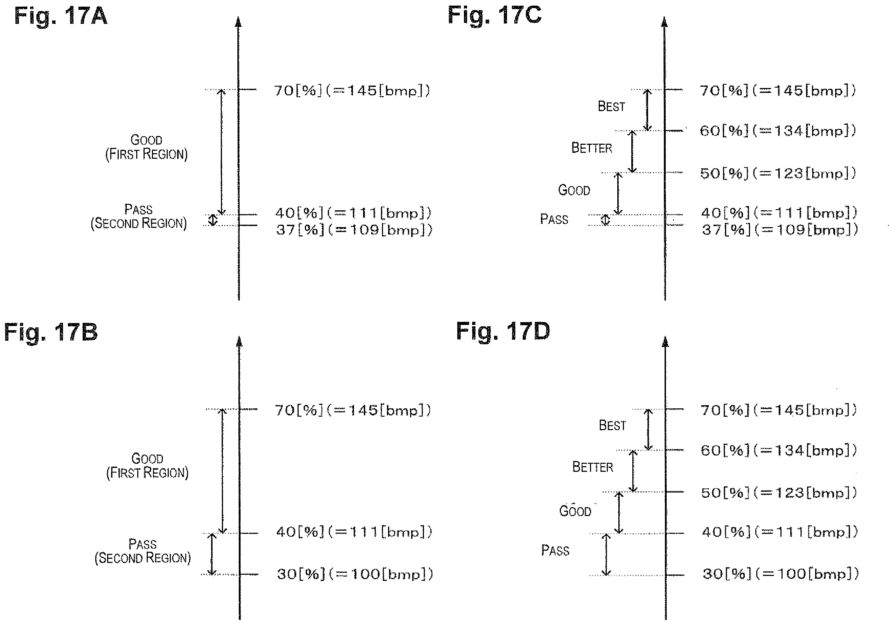

FIGS. 17(A), 17(B), 17(C), and 17(D) show examples of a division of zones relating to exercise intensity. FIGS. 17(A) and 17(C) correspond to, e.g., a zone for the user A in FIG. 14, and FIGS. 17(B) and 17(D) correspond to, e.g., a zone for the user B in FIG. 14. In the example shown in FIG. 17(A), the biological information measuring device divides the zone relating to exercise intensity (i.e., exercise intensity of 37% to 70%) into two regions (i.e., "pass" region and "good" region) according to the first measured value (i.e., a pulse rate of 109 bpm = exercise intensity of 37%). In other words, in addition to an initial first region relating to exercise intensity (i.e., exercise intensity of 40% to 70%: from the first threshold value to the upper limit of the zone), a second region (i.e., exercise intensity of 37% to 40%: from the first measured value to the first threshold value) is added according to the first threshold value (i.e., exercise intensity of 40%). In the example shown in FIG. 17(B), the biological information measuring device divides the zone relating to exercise intensity (i.e., exercise intensity of 30% to 70%) into two regions ("pass" region (i.e., a second region: from the second threshold value to the first threshold value) and "good" region (i.e., a first region: from the first threshold value to the upper limit of the zone)) according to the first threshold value (i.e., exercise intensity of 40%). The initial first region corresponding to the "good" region in FIGS. 17(A) and 17(B) (i.e., exercise intensity of 40% to 70%) can be divided into three regions (i.e., "good" region, "better" region, and "best" region) as shown in FIGS. 17(C) and 17(D). The three regions can respectively be called a first subregion, a second subregion, and a third subregion of the initial first region. Preferably, the first region is divided into two or three subregions, because in an instance in which the first region is too finely divided, it becomes difficult, during exercise, to allocate, e.g., a pulse rate (i.e., the second measured value) into a divided subregion.

The biological information measuring device is thus capable of dividing the zone relating to exercise intensity into a plurality of regions according to the first threshold value. In an instance in which the lower limit of the zone is not corrected according to the first measured value, such as in the example of the zone for user D in FIG. 14, it is possible for the biological information measuring device to only use the zone relating to exercise intensity (i.e., exercise intensity of 40% to 70%) itself. However, the zone for user D may be divided into a plurality of subregions, i.e., three regions ("good" region, "better" region, "best" region), as shown in FIGS. 17(C) and 17(D).

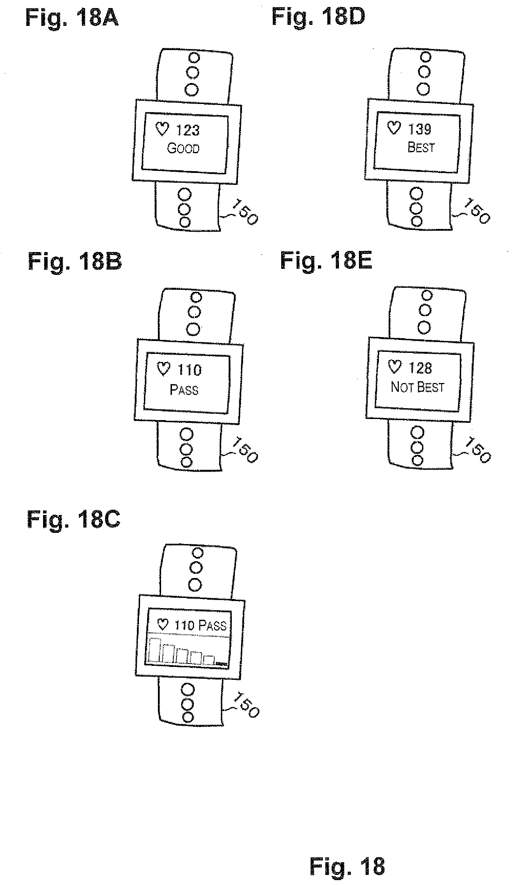

FIGS. 18(A), 18(B), 18(C), 18(D), and 18(E) show examples of a notification of the second measured value.

The notification part 70' (or in a broader sense, the biological information measuring device) is capable of notifying the test subject whether the second measured value relating to exercise intensity determined from biological information during the second exercise period that follows the first exercise period belongs to either one of the first region or the second region. In an instance in which the second region does not exist, the notification part 70' notifies the test subject whether the second measured value belongs to the first region. In an instance in which the first region includes a plurality of subregions, the notification part 70' may notify the test subject of which subregion the second measured value belongs to. Alternatively, the notification part 70' may notify the test subject whether the second measured value belongs to any one of the subregions (e.g., a subregion in which the exercise intensity is the highest among the subregions).

The example shown in FIG. 18(A) shows that the second measured value (e.g., a pulse rate of 123 bpm = exercise intensity of 50%) is equal to or greater than the first threshold value and equal to or less than the upper limit of the zone, and is therefore within, e.g., the "good" region (exercise intensity of 40% to 70%) in FIG. 17(A). The example shown in FIG. 18(B) shows that the second measured value (e.g., a pulse rate of 100 bpm = exercise intensity of 39%) is equal to or greater than the second threshold value or the first measured value and less than the first threshold value, and is therefore within, e.g., the "pass" region (exercise intensity of 37% to 40%) in FIG. 17(A). The "pass" region is a region that has been added to the initial zone; therefore, the test subject is able to increase the pace of walking or another exercise so as to reach a higher region.

The notification part 70' can notify the test subject of which of the regions the second measured value belongs to, as well as notify the test subject of a history of the current position within the zone relating to exercise intensity, as shown in FIG. 18(C). Alternatively, the notification part 70' can notify the test subject of which of the regions the second measured value belongs to, as well as notifying the test subject of the current position within the zone relating to exercise intensity as shown, e.g., in FIG. 16(A). In the example shown in FIG. 18(C), the upper limit (e.g., a pulse rate of 145 bpm = exercise intensity of 70%) and the lower limit (e.g., a pulse rate of 109 bpm = exercise intensity of 37%) of the zone are shown by dotted lines. Also, in the example shown in FIG. 18(C), the history of the current position in the zone relating to exercise intensity shows that the second measured value (i.e., the pulse rate) is gradually decreasing, and a recent current position is represented by a black vertical bar.

As shown in FIGS. 18(D) and 18(E), the notification part 70' can also notify the test subject whether the second measured value belongs to, e.g., the "best" region (or in a broader sense, one subregion among the subregions) shown in FIGS. 17(C) and 17(D). The example shown in FIG. 18(D) shows that the second measured value (e.g., a pulse rate of 139 bpm = exercise intensity of 65%) is equal to or greater than the first threshold value, is equal to or less than the upper limit of the zone, and is within, e.g., the "best" region in FIG. 17(C) among the subregions. In the example shown in FIG. 18(E), the second measured value (e.g., a pulse rate of 128 bpm = exercise intensity of 55%) is less than, e.g., the "best" region in FIG. 17(C) among the subregions. Therefore, the test subject can be notified that the it is not in the "best" region. Being presented with whether it is in a subregion in which the exercise intensity is the highest among the subregions makes it possible for the test subject to perform exercise at a higher exercise intensity.

### 2.3 First configuration example

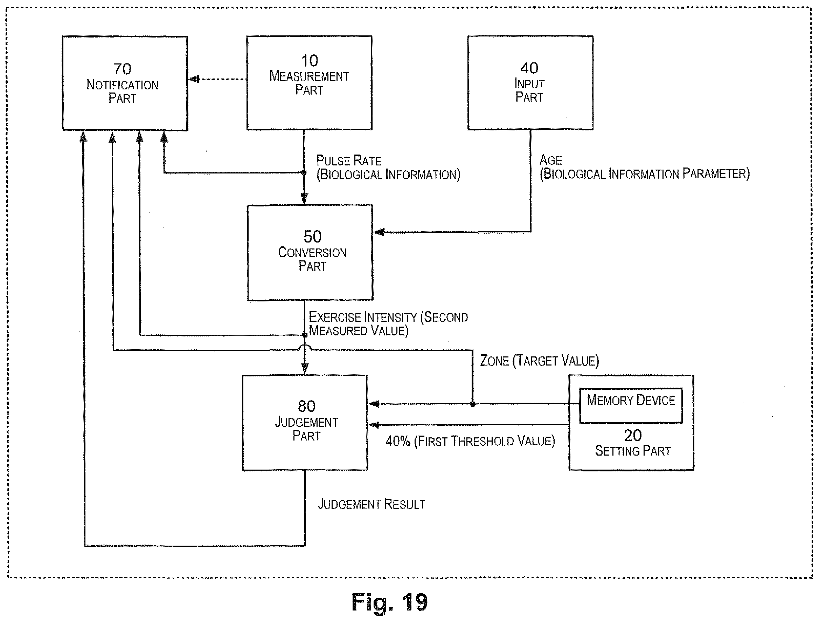

FIG. 19 shows an example of a specific configuration of the biological information measuring device in FIG. 15. In the example shown in FIG. 19, the biological information measuring device is a pulse rate monitor; however, the example of a specific configuration of the biological information measuring device is not limited to that shown in FIG. 19. Structures that are identical to those in the example described above are affixed with the same numerals, and a description of the structures is not provided.

As shown in FIG. 19, the biological information measuring device may further include the input part 40, the conversion part 50, and a judgement part 80. As with the measurement part 10 and the setting part 20, the conversion part 50 and the judgement part 80 may include, e.g., a gate array or another ASIC. All or a some of the functions of the conversion part 50 and the judgement part 80 may also be performed by at least one of the measurement part 10, the setting part 20, and the notification part 70.

The input part 40 functions in a similar manner to that in the first mode. In the second mode, the biological information measuring device may use the age entered into the input part 40 in the first mode. The age, e.g., may also be re-entered as a previously known biological information parameter into the input part 40 during the second mode.

In the example shown in FIG. 19, in the second mode, the conversion part 50 can obtain the exercise intensity according to the pulse rate from the measurement part 10 and the age from the input part 40 using a correspondence relationship such as that shown, e.g., in Equation 1, Equation 2, and FIG. 3. The exercise intensity obtained by the conversion part 50 is the second measured value (i.e., an actual physical performance of the test subject during the second exercise period that follows the first exercise period).

In the example shown in FIG. 19, as with the first mode, e.g., 40% is preset in the setting part 20 as the first exercise intensity threshold value. After the first mode has been performed, the zone (i.e., target value) is stored in, e.g., the memory device. In the example shown in FIG. 19, in the second mode, the judgement part 80 can divide the zone relating to exercise intensity into a plurality of regions according to the first threshold value, and judging which of the regions the second measured value belongs to.

In the example shown in FIG. 19, in the second mode, the notification part 70 can notify the test subject of a result of the comparison performed by the judgement part 80, i.e., which of the regions the second measured value (i.e., the current exercise intensity) belongs to. Also, in the example shown in FIG. 19, in the second mode, the notification part 70 can relate the second measured value (i.e., the current exercise intensity) to the zone relating to exercise intensity, and thereby notify the test subject of the current position in the zone relating to exercise intensity. Also, in the example shown in FIG. 19, the test subject can be notified of the current pulse rate.

### 2.4 Example of operation

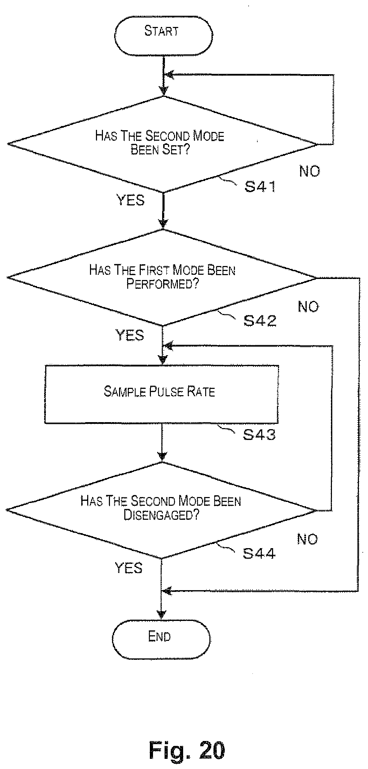

FIG. 20 is a flow chart showing an example of operation of the measurement part 10 in FIG. 19. In the example shown in FIG. 20, the measurement part 10 (or in a broader sense, the biological information measuring device) judges whether the second mode has been set (step S41). In an instance in which the second mode has not been set, the measurement part 10 performs step S41 again. For example, the second mode may be set at the same time as a dedicated operation button or a similar input device for setting the second mode being pressed or otherwise operated.

In the example shown in FIG. 20, the measurement part 10 (or in a broader sense, the biological information measuring device) judges whether the first mode has been performed (step S42). In an instance in which the first mode has not been performed, the test subject must prepare for, e.g., a six-minute walk, and set the biological information measuring device to the first mode. In an instance in which the first mode has been performed, the test subject performs, e.g., a 30-minute walk, and the measurement part 10 can measure the pulse rate during the exercise period in the second mode. The test subject may perform the walk as long as they desire, or may jog or perform another exercise of their choice. The notification part 70 may also issue a message such as "Please start exercising" or otherwise prompt the test subject after the test subject has set the second mode.

In the example shown in FIG. 20, the measurement part 10 (or in a broader sense, the biological information measuring device) samples the pulse rate at predetermined intervals (step S43). The measurement part 10 (or in a broader sense, the biological information measuring device) judges whether the second mode has been disengaged (step S44). In an instance in which the second mode has not been disengaged, the measurement part 10 performs step S43 again. For example, the measurement part 10 can measure the pulse rate in real time, and store the pulse rate, e.g., every 10 seconds until the second mode is disengaged. For example, the second mode can be disengaged at the same time as the dedicated operation button or a similar input device for setting the second mode being pressed or otherwise operated again. Alternatively, the second mode can be disengaged at the same time as a dedicated disengage operation button or a similar input device for disengaging the second mode has been pressed or otherwise operated. The measurement part 10 may measure the time elapsed since the second mode has been set, and automatically disengage the second mode when the elapsed time reaches, e.g., 2 hours.

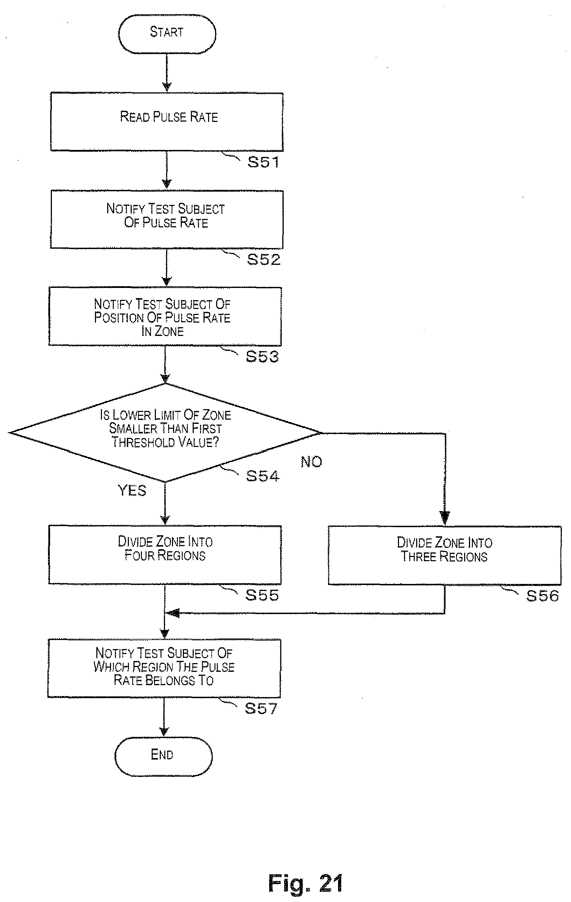

FIG. 21 is a flow chart showing an example of operation of the notification part 70' in FIG. 19. In the example shown in FIG. 21, the notification part 70' (or in a broader sense, the biological information measuring device) reads the pulse rate sampled, e.g., by the measurement part 10, and notifies the test subject of the pulse rate that has been read (steps S51, S52). In an instance in which the pulse rate is stored, e.g., every 10 seconds in step S43 in FIG. 20, the notification part 70 can, in steps S51 and S52 in FIG. 21, renew the pulse rate every 10 seconds and display the current pulse rate as shown, e.g., in FIG. 16(A). The configuration of the notification part 70' is not limited to that shown, e.g., in FIG. 16(A), and the current pulse rate maybe outputted, e.g., as an audible message.

In the example shown in FIG. 21, the notification part 70' (or in a broader sense, the biological information measuring device) notifies the test subject of where the exercise intensity corresponding to the pulse rate sampled, e.g., by the measurement part 10 is positioned in the zone (step S53). The pulse rate may be used as a unit for the second measured value; however, in the example shown in FIGS. 19 and 21, exercise intensity is used as the unit for the second measured value. In an instance in which exercise intensity is used as the unit for the second measured value, the notification part 70' can convert the pulse rate sampled by the measurement part 10 into exercise intensity (i.e., the second measured value), e.g., via the conversion part 50. Although the pulse rate may be used as a unit for the zone relating to exercise intensity, in the example shown in FIGS. 19 and 21, the exercise intensity itself is used as the unit for the zone relating to exercise intensity. Specifically, although a pulse rate zone such as those shown in FIG. 14 may be set into, e.g., the memory device of the setting part 20, an exercise intensity zone is used. In the example shown in FIG. 21, the notification part 70 can use the upper and lower limits of the exercise intensity zone read from, e.g., the setting part 20, as well as an exercise intensity corresponding to the pulse rate sampled by the measurement part 10 (i.e., the second measured value), in Equation 4, and obtain the current position in the zone relating to exercise intensity. The notification part 70 may display the current position as shown, e.g., in FIG. 16(A).

In the example shown in FIG. 21, the notification part 70 (or in a broader sense, the biological information measuring device) judges whether the lower limit of the exercise intensity zone is smaller than the first threshold value (e.g., an exercise intensity of 40%), e.g., via the judgement part 80 and the setting part 20 (step S54). Specifically, the notification part 70 can judge whether the lower limit of the exercise intensity zone (i.e., target value) has been corrected in the first mode. In an instance in which the lower limit of the exercise intensity zone is smaller than the first threshold value and has been corrected, the notification part 70 (or in a broader sense, the biological information measuring device) divides the exercise intensity zone into four regions ("pass" region, "good" region, "better" region, and "best" region), e.g., via the judgement part 80, as shown, e.g., in FIG. 17(C) (step S55). In an instance in which the lower limit of the exercise intensity zone is equal to or greater than the first threshold value and has not been corrected, the notification part 70' divides the exercise intensity zone into three regions, e.g., via the judgement part 80 (step S56). In an instance in which the lower limit of the exercise intensity zone has not been corrected, a "pass" region such as that shown, e.g., in FIG. 17(C) does not exist. Therefore, the first region (exercise intensity of 40% to 70%) can be divided into three regions (i.e., "good" region, "best" region, and "best" region).

In the example shown in FIG. 21, the notification part 70' (or in a broader sense, the biological information measuring device) judges which of the regions the exercise intensity corresponding to the pulse rate sampled by measurement part 10 (i.e., the second measured value) belongs to, e.g., via the judgement part 80, and notifies the test subject of a result of the judgement (step S57). The notification part 70' may display the result of the judgement as shown, e.g., in FIG. 18(B). In an instance in which the result of the judgement indicates that it is in the "pass" region, the notification part 70' (or in a broader sense, the biological information measuring device) may issue an assistance message such as "Increase the exercise pace somewhat to enter the "good" region" as, e.g., an audible message. In an instance in which the result of the judgement shows that it is not in any region, the notification part 70' (or in a broader sense, the biological information measuring device) may issue a warning such as "The exercise pace is not appropriate" as, e.g., an audible message. The warning may be outputted as an alarm sound or another audible signal, as a red light or another light signal, or as a vibration.

### 2.5 Example of modification

Assuming that, in the example shown in FIG. 19, a pulse rate zone is set in the memory device of the setting part 20 as shown in FIG. 4, the notification part 70' in FIG. 19 may treat the pulse rate sampled by the measurement part 10 itself as the second measured value. Specifically, the notification part 70' in FIG. 19 may be modified; the upper and lower limits of the pulse rate zone read from, e.g., the setting part 20, and the pulse rate sampled by the measurement part 10 (i.e., the second measured value) used in Equation 4; and the current position in the zone relating to exercise intensity obtained. The unit handled by the notification part 70' can thus be the pulse rate or exercise intensity.

### 3 Electronic device

The pulse rate monitor or another biological information measuring device may be installed in a clock, a mobile phone, a pager, a PC, or another electronic device; or may be combined with the electronic device. A part of the biological information measuring device, such as the correction part 30, the conversion part 50, and the comparison part 60 in FIG. 2 and the judgement part 80 and other components in FIG. 19, may include an MPU (i.e., a microprocessing unit) of an electronic device installed with, e.g., a biological information detector.

Although the present embodiment has been described in detail above, persons skilled in the art should be able to easily understand that various modifications are possible without substantially departing from the scope and effects of the invention. Accordingly, all of such examples of modifications are to be included in the scope of the invention. For example, terms stated at least once together with different terms having broader sense or identical sense in the specification or drawings may be replaced with those different terms in any and all locations of the specification or drawings.

### Claims

1.    A biological information measuring device comprising:

a setting part (20) for setting a target value relating to exercise intensity;
a measurement part (10) for measuring biological information that can be converted to exercise intensity;
a correction part (30) for correcting the target value according to a first measured value relating to exercise intensity, the first measured value being determined from biological information measured by the measurement part during a first exercise period; and
**characterised by** further comprising a first notification part (70) for notifying a test subject of an ideal exercise pace for the first exercise period and a current pace.

2. The biological information measuring device according to Claim 1, wherein
the correction part (30) is arranged to obtain the first measured value according to a previously known biological information parameter.

3. The biological information measuring device according to Claim 1, wherein
in an instance in which the first measured value is smaller than a first threshold value, the correction part (30) is arranged to correct the target value to the first measured value.

4. The biological information measuring device according to Claim 3, wherein
in an instance in which the first measured value is equal to or less than a second threshold value that is smaller than the first threshold value, the correction part (30) is arranged to correct the target value to the second threshold value.

5. The biological information measuring device according to Claim 1, wherein
the target value is a lower limit of a zone relating to exercise intensity, and
the biological information measuring device further includes a second notification part (70') for notifying the test subject of a current position within the zone according to a second measured value relating to exercise intensity determined from biological information during a second exercise period that follows the first exercise period.

6. The biological information measuring device according to Claim 3, wherein:

   the target value is a lower limit of a zone relating to exercise intensity;
   the zone has a first region from the first threshold value to an upper limit of the zone;
   the first region is divided into a plurality of subregions; and
   the biological information measuring device further includes a notification part (70, 70') arranged to notify the test subject whether a second measured value relating to exercise intensity determined from biological information during a second exercise period that follows the first exercise period belongs to one of the subregions of the first region.

7. The biological information measuring device according to Claim 6, wherein:

   the one of the subregions is a subregion in which the exercise intensity is the highest among the subregions.

8. The biological information measuring device according to Claim 4, wherein:

   the target value is a lower limit of a zone relating to exercise intensity;
   the zone is divided into a first region from the first threshold value to an upper limit of the zone and a second region from the second threshold value or the first measured value to the first threshold value; and
   the biological information measuring device further includes a notification part (70,70') arranged to notify the test subject whether a second measured value relating to exercise intensity determined from biological information during a second exercise period that follows the first exercise period belongs to one of either the first region or the second region.

9. The biological information measuring device according to Claim 3, wherein, in the instance in which the first measured value is smaller than the first threshold value:

   the target value is a lower limit of a zone relating to exercise intensity;
   the zone is divided into a first region from the first threshold value to an upper limit of the zone and a second region from the first measured value to the first threshold value; and
   the biological information measuring device further includes a notification part (70,70') arranged to notify the test subject whether a second measured value relating to exercise intensity determined from biological information during a second exercise period that follows the first exercise period belongs to one of either the first region or the second region.

10. The biological information measuring device according to Claim 1, wherein
the measurement part (10) is arranged to measure a pulse rate or a heart rate as the biological information, and
the correction part (30) is arranged to correct the target value according to the first measured value determined from the pulse rate or the heart rate measured by the measurement part (10) during the first exercise period.

**Patentansprüche**

1. Vorrichtung zum Messen biologischer Informationen, umfassend:

   einen Einstellungteil (20) zum Einstellen eines Zielwerts bezogen auf Übungsintensität;
   einen Messungsteil (10) zum Messen biologischer Informationen, die zu Übungsintensität umgewandelt werden können;
   einen Korrekturteil (30) zum Korrigieren des Zielwerts gemäß einem ersten gemessenen Wert bezogen auf Übungsintensität, wobei der erste gemessene Wert aus biologischen Informationen bestimmt wird, die von dem Messungsteil während einer ersten Übungsdauer gemessen werden; und
   **dadurch gekennzeichnet, dass** sie weiter einen ersten Benachrichtigungsteil (70) zum Benachrichtigen einer Versuchsperson über ein ideales Übungstempo für die erste Übungsdauer und ein aktuelles Tempo umfasst.

2. Vorrichtung zum Messen biologischer Informationen nach Anspruch 1, wobei der Korrekturteil (30) eingerichtet ist, um den ersten gemessenen Wert gemäß einem zuvor bekannten biologischen Informationsparameter zu erhalten.

3. Vorrichtung zum Messen biologischer Informationen nach Anspruch 1, wobei in einem Fall, in dem der erste gemessene Wert kleiner als ein erster Schwellenwert ist, der Korrekturteil (30) eingerichtet ist, um den Zielwert auf den ersten gemessenen Wert zu korrigieren.

4. Vorrichtung zum Messen biologischer Informationen nach Anspruch 3, wobei in einem Fall, in dem der erste gemessene Wert gleich oder kleiner als ein zweiter Schwellenwert ist, der kleiner als der erste Schwellenwert ist, der Korrekturteil (30) eingerichtet ist, um den Zielwert auf den zweiten Schwellenwert zu korrigieren.

5. Vorrichtung zum Messen biologischer Informationen nach Anspruch 1, wobei der Zielwert eine Untergrenze eines Bereichs bezogen auf Übungsintensität ist und die Vorrichtung zum Messen biologischer Informationen weiter einen zweiten Benachrichtigungsteil (70') zum Benachrichtigen der Versuchsperson über eine aktuelle Position innerhalb des Bereichs gemäß einem zweiten gemessenen Wert bezogen auf Übungsintensität, bestimmt aus biologischen Informationen während einer zweiten Übungsdauer, die auf die erste Übungsdauer folgt, einschließt.

6. Vorrichtung zum Messen biologischer Informationen nach Anspruch 3, wobei:

   der Zielwert eine Untergrenze eines Bereichs bezogen auf Übungsintensität ist;
   der Bereich eine erste Region von dem ersten Schwellenwert zu einer Obergrenze des Bereichs aufweist;
   die erste Region in eine Vielzahl von Unterregionen aufgeteilt ist; und
   die Vorrichtung zum Messen biologischer Informationen weiter einen Benachrichtigungsteil (70, 70') einschließt, der eingerichtet ist, um die Versuchsperson zu benachrichtigen, ob ein zweiter gemessener Wert bezogen auf Übungsintensität, bestimmt aus biologischen Informationen während einer zweiten Übungsdauer, die auf die erste Übungsdauer folgt, zu einer der Unterregionen der ersten Region gehört.

7. Vorrichtung zum Messen biologischer Informationen nach Anspruch 6, wobei:

   die eine der Unterregionen eine Unterregion ist, in der die Übungsintensität die höchste unter den Unterregionen ist.

8. Vorrichtung zum Messen biologischer Informationen nach Anspruch 4, wobei:

   der Zielwert eine Untergrenze eines Bereichs bezogen auf Übungsintensität ist;
   der Bereich in eine erste Region von dem ersten Schwellenwert zu einer Obergrenze des Bereichs und eine zweite Region von dem zweiten Schwellenwert oder dem ersten gemessenen Wert zu dem ersten Schwellenwert aufgeteilt ist; und
   die Vorrichtung zum Messen biologischer Informationen weiter einen Benachrichtigungsteil (70, 70') einschließt, der eingerichtet ist, um die Versuchsperson zu benachrichtigen, ob ein zweiter gemessener Wert bezogen auf Übungsintensität, bestimmt aus biologischen Informationen während einer zweiten Übungsdauer, die auf die erste Übungsdauer folgt, zu einer von entweder der ersten Region oder der zweiten Region gehört.

9. Vorrichtung zum Messen biologischer Informationen nach Anspruch 3, wobei in dem Fall, in dem der erste gemessene Wert kleiner als der erste Schwellenwert ist:

   der Zielwert eine Untergrenze eines Bereichs bezogen auf Übungsintensität ist;
   der Bereich in eine erste Region von dem ersten Schwellenwert zu einer Obergrenze des Bereichs und eine zweite Region von dem ersten gemessenen Wert zu dem ersten Schwellenwert aufgeteilt ist; und
   die Vorrichtung zum Messen biologischer Informationen weiter einen Benachrichtigungsteil (70, 70') einschließt, der eingerichtet ist, um die Versuchsperson zu benachrichtigen, ob ein zweiter gemessener Wert bezogen auf Übungsintensität, bestimmt aus biologischen Informationen während einer zweiten Übungsdauer, die auf die erste Übungsdauer folgt, zu einer von entweder der ersten Region oder der zweiten Region gehört.

10. Vorrichtung zum Messen biologischer Informationen nach Anspruch 1, wobei
    der Messungsteil (10) eingerichtet ist, um eine Pulsfrequenz oder eine Herzfrequenz als die biologischen Informationen zu messen, und
    der Korrekturteil (30) eingerichtet ist, um den Zielwert gemäß dem ersten gemessenen Wert, bestimmt aus der Pulsfrequenz oder der Herzfrequenz, gemessen von dem Messungsteil (10) während der ersten Übungsdauer, zu korrigieren.

**Revendications**

1. Dispositif de mesure d'informations biologiques comprenant :

   une partie de réglage (20) pour régler une valeur cible concernant une intensité d'exercice ;
   une partie de mesure (10) pour mesurer des informations biologiques qui peuvent être converties en intensité d'exercice ;
   une partie de correction (30) pour corriger la valeur cible en fonction d'une première valeur mesurée concernant l'intensité d'exercice, la première valeur mesurée étant déterminée à partir d'informations biologiques mesurées par la partie de mesure durant une première période d'exercice ; et
   **caractérisé en ce qu'**il comprend en outre une première partie de notification (70) pour notifier à un sujet de test une cadence d'exercice idéale pour la première période d'exercice et une cadence actuelle.

2. Dispositif de mesure d'informations biologiques selon la revendication 1, dans lequel
   la partie de correction (30) est agencée pour obtenir la première valeur mesurée en fonction d'un paramètre d'informations biologiques préalablement connu.

3. Dispositif de mesure d'informations biologiques selon la revendication 1, dans lequel
   dans un cas dans lequel la première valeur mesurée est inférieure à une première valeur de seuil, la partie de correction (30) est agencée pour corriger la valeur cible pour la première valeur mesurée.

4. Dispositif de mesure d'informations biologiques selon la revendication 3, dans lequel
   dans un cas dans lequel la première valeur mesurée est inférieure ou égale à une seconde valeur de seuil qui est inférieure à la première valeur de seuil, la partie de correction (30) est agencée pour corriger la valeur cible pour la seconde valeur de seuil.

5. Dispositif de mesure d'informations biologiques selon la revendication 1, dans lequel
   la valeur cible est une limite inférieure d'une zone concernant une intensité d'exercice, et
   le dispositif de mesure d'informations biologiques comporte en outre une seconde partie de notification (70') pour notifier au sujet de test une position actuelle à l'intérieur de la zone en fonction d'une seconde valeur mesurée concernant une intensité d'exercice déterminée à partir d'informations biologiques durant une seconde période d'exercice qui suit la première période d'exercice.

6. Dispositif de mesure d'informations biologiques selon la revendication 3, dans lequel :

   la valeur cible est une limite inférieure d'une zone concernant une intensité d'exercice ;
   la zone a une première région de la première valeur de seuil à une limite supérieure de la zone ;
   la première région est divisée en une pluralité de sous-régions ; et
   le dispositif de mesure d'informations biologiques comporte en outre une partie de notification (70, 70') agencée pour notifier au sujet du test si une seconde valeur mesurée concernant une intensité d'exercice déterminée à partir d'informations biologiques durant une seconde période d'exercice qui suit la première période d'exercice appartient à l'une des sous-régions de la première région.

7. Dispositif de mesure d'informations biologiques selon la revendication 6, dans lequel :

l'une des sous-régions est une sous-région dans laquelle l'intensité d'exercice est la plus élevée parmi les sous-régions.

8. Dispositif de mesure d'informations biologiques selon la revendication 4, dans lequel :

la valeur cible est une limite inférieure d'une zone concernant une intensité d'exercice ;
la zone est divisée en une première région de la première valeur de seuil à une limite supérieure de la zone et une seconde région de la seconde valeur de seuil ou de la première valeur mesurée à la première valeur de seuil ; et
le dispositif de mesure d'informations biologiques comporte en outre une partie de notification (70, 70') agencée pour notifier au sujet du test si une seconde valeur mesurée concernant une intensité d'exercice déterminée à partir d'informations biologiques durant une seconde période d'exercice qui suit la première période d'exercice appartient à l'une de la première région ou de la seconde région.

9. Dispositif de mesure d'informations biologiques selon la revendication 3, dans lequel, dans le cas dans lequel la première valeur mesurée est inférieure à la première valeur de seuil :

la valeur cible est une limite inférieure d'une zone concernant une intensité d'exercice ;
la zone est divisée en une première région de la première valeur de seuil à une limite supérieure de la zone et une seconde région de la première valeur mesurée à la première valeur de seuil ; et
le dispositif de mesure d'informations biologiques comporte en outre une partie de notification (70, 70') agencée pour notifier au sujet du test si une seconde valeur mesurée concernant une intensité d'exercice déterminée à partir d'informations biologiques durant une seconde période d'exercice qui suit la première période d'exercice appartient à l'une ou l'autre de la première région ou de la seconde région.

10. Dispositif de mesure d'informations biologiques selon la revendication 1, dans lequel
la partie de mesure (10) est agencée pour mesurer une fréquence du pouls ou une fréquence cardiaque en tant qu'informations biologiques, et
la partie de correction (30) est agencée pour corriger la valeur cible en fonction de la première valeur mesurée déterminée à partir de la fréquence du pouls ou de la fréquence cardiaque mesurée par la partie de mesure (10) durant la première période d'exercice.

**10**
MEASUREMENT
PART

**20**
SETTING
PART

TARGET
VALUE

BIOLOGICAL INFORMATION
(FIRST MEASURED VALUE)

**30**
CORRECTION
PART

# Fig. 1

Fig. 2

| AGE (YEARS) | RESTING PULSE (bpm) |
|:---:|:---:|
| 0 | 70 |
| 5 | 70 |
| 10 | 70 |
| 15 | 70 |
| 20 | 70 |
| 25 | 69 |
| 30 | 68 |
| 35 | 67 |
| 40 | 66 |
| 45 | 65 |
| 50 | 64 |
| 55 | 63 |
| 60 | 62 |
| 65 | 61 |
| 70 | 60 |
| 75 | 60 |
| 80 | 60 |
| 85 | 60 |
| 90 | 60 |
| 95 | 60 |

# Fig. 3

**Fig. 4**

EP 2 351 519 B1

Fig. 5

23

Fig. 6

START

READ EXERCISE INTENSITY

S21

CONVERT EXERCISE INTENSITY TO
PULSE RATE (TARGET VALUE)

S22

SET PULSE RATE (TARGET VALUE)

S23

DOES PULSE RATE
(TARGET VALUE)
NEED CORRECTING?

NO

S24

YES

SET CORRECTED PULSE RATE

S25

END

Fig. 7

| AGE (YEARS) | PULSE RATE TARGET VALUE (bpm) (EXERCISE INTENSITY = 40%) |
|---|---|
| 25 | 119 |
| 26 | 119 |
| 27 | 119 |
| 28 | 118 |
| 29 | 118 |
| 30 | 117 |
| 31 | 116 |
| 32 | 116 |
| 33 | 116 |
| 34 | 115 |
| 35 | 114 |
| 36 | 114 |
| 37 | 113 |
| 38 | 113 |
| 39 | 113 |
| 40 | 112 |
| 41 | 111 |
| 42 | 111 |
| 43 | 110 |
| 44 | 110 |
| 45 | 109 |
| 46 | 109 |
| 47 | 108 |
| 48 | 108 |
| 49 | 107 |
| 50 | 106 |
| 51 | 106 |
| 52 | 106 |
| 53 | 105 |
| 54 | 105 |
| 55 | 104 |
| 56 | 103 |
| 57 | 103 |
| 58 | 103 |
| 59 | 102 |
| 60 | 101 |

## Fig. 8

START

CONVERT PULSE RATE (BIOLOGICAL INFORMATION) TO EXERCISE INTENSITY (FIRST MEASURED VALUE)

S31

IS EXERCISE INTENSITY (FIRST MEASURED VALUE) SMALLER THAN FIRST THRESHOLD VALUE?

S32

NO

YES

IS EXERCISE INTENSITY (FIRST MEASURED VALUE) GREATER THAN SECOND THRESHOLD VALUE?

S33

NO

YES

CONVERT EXERCISE INTENSITY (FIRST MEASURED VALUE) TO PULSE RATE (CORRECTIVE PULSE RATE)

S34

CONVERT SECOND THRESHOLD VALUE TO PULSE RATE (CORRECTIVE PULSE RATE)

S35

INSTRUCT CORRECTION OF PULSE RATE (TARGET VALUE)

S36

END

Fig. 9

| Test Subject | First Measured Value (%) | First Threshold Value (%) | Second Threshold Value (%) | Pulse Rate Target Value (bpm) (Exercise Intensity = 40%) | Corrective Pulse Rate (bpm) |
|---|---|---|---|---|---|
| User A | 37 | 40 | 30 | 111 | 109 |
| User B | 20 | 40 | 30 | 111 | 100 |
| User C | 21 | 40 | 30 | 114 | 102 |
| User D | 45 | 40 | 30 | 114 | — |

**Fig. 10**

EP 2 351 519 B1

```
┌──────────────┐        ┌──────────────┐
│      10      │        │      40      │
│ MEASUREMENT  │        │    INPUT     │
│     PART     │        │     PART     │
└──────────────┘        └──────────────┘
```

PULSE RATE
(BIOLOGICAL INFORMATION)
(FIRST MEASURED VALUE)

AGE
(BIOLOGICAL INFORMATION PARAMETER)

CORRECTIVE EXERCISE INTENSITY
(TARGET VALUE)

```
┌──────────────┐
│      50      │
│  CONVERSION  │
│     PART     │
└──────────────┘
```

40%, 30%

PULSE RATE (First Threshold Value)
PULSE RATE (Second Threshold Value)

CORRECTIVE PULSE RATE

```
┌──────────────┐            ┌──────────────────┐
│      60      │            │        20        │
│  COMPARISON  │◄───────────│   SETTING PART   │
│     PART     │            │  ┌────────────┐  │
└──────────────┘            │  │   MEMORY   │  │ 40%
                            │  │   DEVICE   │  │ (TARGET VALUE)
                            │  └────────────┘  │
                            └──────────────────┘
```

COMPARISON RESULT

```
┌──────────────┐
│      30      │
│  CORRECTION  │
│     PART     │
└──────────────┘
```

**Fig. 11**

Fig. 12

| Test Subject | First Measured Value (%) | First Threshold Value (%) | Second Threshold Value (%) | Exercises Intensity Target Value (%) | Corrective Exercise Intensity (%) |
|---|---|---|---|---|---|
| User A | 37 | 40 | 30 | 40 | 37 |
| User B | 20 | 40 | 30 | 40 | 30 |
| User C | 21 | 40 | 30 | 40 | 30 |
| User D | 45 | 40 | 30 | 40 | — |

**Fig. 13A**

| Test Subject | First Measured Value (bmp) | First Threshold Value (bmp) | Second Threshold Value (bmp) | Exercises Intensity Target Value (%) | Corrective Exercise Intensity (%) |
|---|---|---|---|---|---|
| User A | 108 | 111 | 100 | 40 | 37 |
| User B | 89 | 111 | 100 | 40 | 30 |
| User C | 92 | 114 | 102 | 40 | 30 |
| User D | 120 | 114 | 102 | 40 | — |

**Fig. 13B**

| Test Subject | First Measured Value (bmp) | First Threshold Value (bmp) | Second Threshold Value (bmp) | Pulse Rate Target Value (bmp) (Exercise Intensity = 40%) | Corrective Pulse Rate (bmp) |
|---|---|---|---|---|---|
| User A | 108 | 111 | 100 | 111 | 108 |
| User B | 89 | 111 | 100 | 111 | 100 |
| User C | 92 | 114 | 102 | 114 | 102 |
| User D | 120 | 114 | 102 | 114 | — |

**Fig. 13C**

31

| TEST SUBJECT | FIRST MEASURED VALUE (%) | PULSE RATE TARGET VALUE (bpm) (EXERCISE INTENSITY = 40%) | CORRECTIVE PULSE RATE (bpm) (CORRECTIVE LOWER LIMIT) | PULSE RATE TARGET VALUE (bpm) (EXERCISE INTENSITY = 70%) | PULSE RATE ZONE (bpm) |
|---|---|---|---|---|---|
| USER A | 37 | 111 | 109 | 145 | 109～145 |
| USER B | 20 | 111 | 100 | 145 | 100～145 |
| USER C | 21 | 114 | 102 | 150 | 102～150 |
| USER D | 45 | 114 | — | 150 | 114～150 |

## Fig. 14

EP 2 351 519 B1

```
          ┌─────────────┐
          │     10      │
          │ MEASUREMENT │          ┌──────────┐
          │    PART     │          │    20    │           ZONE
          └─────────────┘          │ SETTING  │ ────────► (TARGET VALUE)
                 │                  │   PART   │
                 ▼                  └──────────┘
       BIOLOGICAL INFORMATION
      (SECOND MEASURED VALUE)
                 │
                 ▼
          ┌─────────────┐
          │     70      │
          │NOTIFICATION │
          │    PART     │
          └─────────────┘
```

# Fig. 15

## Fig. 16A

## Fig. 16B

## Fig. 17A

GOOD (FIRST REGION): 70[%] (=145[bmp])

PASS (SECOND REGION): 40[%] (=111[bmp]), 37[%] (=109[bmp])

## Fig. 17B

GOOD (FIRST REGION): 70[%] (=145[bmp])

PASS (SECOND REGION): 40[%] (=111[bmp]), 30[%] (=100[bmp])

## Fig. 17C

BEST: 70[%] (=145[bmp])

BETTER: 60[%] (=134[bmp])

GOOD: 50[%] (=123[bmp])

PASS: 40[%] (=111[bmp]), 37[%] (=109[bmp])

## Fig. 17D

BEST: 70[%] (=145[bmp])

BETTER: 60[%] (=134[bmp])

GOOD: 50[%] (=123[bmp])

PASS: 40[%] (=111[bmp]), 30[%] (=100[bmp])

**Fig. 18A**

**Fig. 18D**

**Fig. 18B**

**Fig. 18E**

**Fig. 18C**

**Fig. 18**

Fig. 19

START

HAS THE SECOND MODE
BEEN SET?

NO

S41

YES

HAS THE FIRST MODE BEEN
PERFORMED?

NO

S42

YES

SAMPLE PULSE RATE

S43

HAS THE SECOND MODE BEEN
DISENGAGED?

NO

S44

YES

END

Fig. 20

START

READ PULSE RATE

S51

NOTIFY TEST SUBJECT
OF PULSE RATE

S52

NOTIFY TEST SUBJECT OF
POSITION OF PULSE RATE
IN ZONE

S53

IS LOWER LIMIT OF ZONE
SMALLER THAN FIRST
THRESHOLD VALUE?

S54

NO

YES

DIVIDE ZONE INTO
FOUR REGIONS

S55

DIVIDE ZONE INTO
THREE REGIONS

S56

NOTIFY TEST SUBJECT OF
WHICH REGION THE PULSE
RATE BELONGS TO

S57

END

Fig. 21

**REFERENCES CITED IN THE DESCRIPTION**

**Patent documents cited in the description**

- JP 3421738 B

- US 20040122333 A1